# EUROPEAN PATENT APPLICATION

(11) **EP 4 807 684 A1**
(43) Date of publication of application: **16.09.2026**
(21) Application number: 26163897.7
(22) Date of filing: 11.03.2026
(51) Int. Cl.: G06T 15/50, G06T 19/20

(54) **ULTRASONIC DIAGNOSTIC APPARATUS, MEDICAL IMAGE PROCESSING APPARATUS AND PROGRAM**

(30) Priority: 11.03.2025 JP 2025038375
(71) Applicant: Canon Medical Systems Corporation, Otawara-shi, Tochigi 324-0036 (JP)
(72) Inventor: KUGA, Itsuki, Otawara-shi, 324-0036 (JP)
(74) Representative: Marks & Clerk LLP

(57) **Abstract**

An ultrasonic diagnostic apparatus according to an embodiment includes a first acquisition unit, a second acquisition unit, and a generation unit. The first acquisition unit acquires a first image on which surface rendering has been executed on three-dimensional ultrasonic data related to a subject. The second acquisition unit acquires a light attenuation coefficient corresponding to the three-dimensional ultrasonic data. The generation unit generates a rendering image based on the first image and the light attenuation coefficient.

## Description

### CROSS REFERENCE TO RELATED APPLICATIONS

This application is based upon and claims the benefit of priority from the prior Japanese Patent Application No. 2025-038375, filed on March 11, 2025, the entire contents of which are incorporated herein by reference.

### FIELD

Embodiments described in the present specification and drawings relate to an ultrasonic diagnostic apparatus, a medical image processing apparatus and a program.

### BACKGROUND

Hitherto, a technique for acquiring three-dimensional ultrasonic data using a 3D/4D mode, generating a rendering image, and displaying the generated rendering image is known in ultrasonic diagnostic apparatuses. In recent years, a concept of a light source is adopted, and a global illumination rendering image is generated by global illumination rendering processing, which is a rendering method taking color attenuation and reflection into consideration.

On the other hand, in diagnosis using an ultrasonic image diagnostic apparatus, a surface rendering image according to surface rendering processing, which is displayed by applying a color map using shading processing calculated from luminance of ultrasonic data or a local gradient, is also used. Such a surface rendering image is often used because, although it is not shaded, it easily has a contour boundary and can be displayed without requiring parameter adjustment after generating the surface rendering image. On the other hand, since this surface rendering image does not have a depth factor, the surface rendering image may have a poor depth feeling and stereoscopic effect. For this reason, a technique for providing a depth feeling and a stereoscopic effect to a surface rendering image is known. For example, as a technique for providing a depth feeling or a stereoscopic effect to a surface rendering image, there is a technique of blurring an image, darkening a color, or changing a color toward a deeper side of a space of ultrasonic data, such as depth cueing processing or fog processing. In particular, for three-dimensional ultrasonic data, color depth cueing in which depth cueing processing is colorized is often used.

However, when color depth cueing is used in the surface rendering processing, a certain degree of depth feeling is obtained, but the color depth cueing does not contribute to a local stereoscopic effect. When shading is used in the surface rendering processing, a local stereoscopic effect can be obtained, but the shading does not contribute to a stereoscopic effect in a wide area. When global illumination rendering processing is used, a high stereoscopic effect, depth feeling, and real texture are obtained due to color attenuation and shadow. However, depending on a position of a light source, a portion that is deeply shadowed on a global illumination image is generated. Thus, visibility of the portion that is shadowed on the global illumination image may be reduced. For this reason, it is desired to generate a rendering image capable of securing visibility of a shadow portion while imparting a shadow effect.

### SUMMARY OF INVENTION

An ultrasonic diagnostic apparatus comprising
processing circuitry configured to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.

The processing circuitry may be configured to acquire a second image on which global illumination has been executed on the three-dimensional ultrasonic data and acquire the light attenuation coefficient based on the second image.

The processing circuitry may be configured to generate a plurality of color images by applying a plurality of color maps to the first image subjected to the surface rendering.

The processing circuitry may be configured to generate the rendering image by combining the plurality of color images based on the light attenuation coefficient.

The processing circuitry may be configured to:
generate a first color image by applying a first color map to the first image; and/or
generate a second color image by applying a second color map different from the first color map to the first image.

The processing circuitry may be configured to generate the rendering image by combining the first color image and the second color image using the light attenuation coefficient.

The ultrasonic diagnostic apparatus may comprise a combination algorithm memory configured to store a plurality of combination algorithms for combining the first color image and the second color image.

The processing circuitry may be configured to select a combination algorithm to be used from among the plurality of combination algorithms stored in the combination algorithm memory in accordance with input information input by a user and/or preset information that has been preset, and combine the first color image and the second color image by using the selected combination algorithm together with the light attenuation coefficient.

The processing circuitry may be configured to combine the first color image and the second color image by linear interpolation using the light attenuation coefficient.

The processing circuitry may be configured to:
execute photon mapping processing as the global illumination on the three-dimensional ultrasonic data; and
acquire the second image based on a photon map that is a map related to attenuation of light generated by executing the photon mapping processing.

The processing circuitry may be configured to set an attenuation transfer function for determining a degree of attenuation of light.

The processing circuitry may be configured to acquire the photon map using the attenuation transfer function in the photon mapping processing.

The processing circuitry may be configured to set a reflection transfer function for determining a degree of reflection of light by determining luminance and/or color.

The processing circuitry may be configured to acquire the second image based on the reflection transfer function together with the photon map.

The processing circuitry may be configured to execute color depth cueing processing on the first image.

The processing circuitry may be configured to control a display unit to display the rendering image.

The processing circuitry may be configured to apply, to the second image, a coefficient conversion transfer function for converting the second image into the light attenuation coefficient to convert the second image into the light attenuation coefficient.

The ultrasonic diagnostic apparatus may comprise a texture memory configured to store a texture having a plurality of channels.

The texture may be configured to be able to store image data based on three-dimensional ultrasonic data in each of the plurality of channels.

A medical image processing apparatus comprising
processing circuitry configured to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.

A program causing a computer to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.

### BRIEF DESCRIPTION OF THE DRAWINGS

FIG. 1 is a block diagram illustrating an example of a configuration of an ultrasonic diagnostic apparatus according to a first embodiment.
FIG. 2 is a diagram for describing a method of generating a composite image.
FIG. 3 is a flowchart for describing composite image generation processing executed in the ultrasonic diagnostic apparatus according to the first embodiment.
FIG. 4 is a diagram illustrating an example of a surface rendering image according to the first embodiment.
FIG. 5 is a diagram illustrating an example of a light attenuation image in the first embodiment.
FIG. 6 is a diagram illustrating an example of a composite image in which a light source is in front in the first embodiment.
FIG. 7 is a diagram illustrating an example of a composite image in which the light source is in a right direction in the first embodiment.
FIG. 8 is a diagram illustrating an example of a composite image in which the light source is in an upper right direction in the first embodiment.
FIG. 9 is a diagram illustrating an example of a global illumination rendering image in which the light source is in the upper right direction, the global illumination rendering image being generated by global illumination rendering processing in the first embodiment.
FIG. 10 is a diagram illustrating an example of a color depth cueing image generated by color depth cueing processing in the first embodiment.
FIG. 11 is a flowchart for describing composite image generation processing executed in an ultrasonic diagnostic apparatus according to a second embodiment.
FIG. 12 is a diagram illustrating an example of a surface rendering image according to the second embodiment.
FIG. 13 is a diagram illustrating an example of a composite image in which a light source is in front in the second embodiment.
FIG. 14 is a diagram illustrating an example of a color depth cueing image generated by color depth cueing processing in the second embodiment.
FIG. 15 is a diagram illustrating an example of a composite image in which a light source is in a right direction.
FIG. 16A is a diagram illustrating an example of a composite image in which a light source is on an upper left side in the second embodiment.
FIG. 16B is a diagram illustrating an example of a composite image in which the light source is on an upper right side in the second embodiment.
FIG. 17 is a block diagram illustrating an example of a configuration of an ultrasonic diagnostic apparatus according to a third embodiment.
FIG. 18 is a flowchart for describing composite image generation processing executed in the ultrasonic diagnostic apparatus according to the third embodiment.
FIG. 19A is a diagram illustrating an example of a composite image generated using a base color image having been subjected to color depth cueing processing in which a light source is in an upper left direction in the third embodiment.
FIG. 19B is a diagram illustrating an example of a composite image generated using a base color image having not been subjected to the color depth cueing processing in which the light source is in an upper left direction in the third embodiment.
FIG. 20A is a diagram illustrating an example of a composite image generated using a base color image having been subjected to the color depth cueing processing in which the light source is in a lower left direction in the third embodiment.
FIG. 20B is a diagram illustrating an example of a composite image generated using a base color image having not been subjected to the color depth cueing processing in which the light source is in a lower left direction in the third embodiment.
FIG. 21A is a diagram illustrating an example of a composite image generated using a base color image having been subjected to the color depth cueing processing in which the light source is in an upper right direction in the third embodiment.
FIG. 21B is a diagram illustrating an example of a composite image generated using a base color image having not been subjected to the color depth cueing processing in which the light source is in an upper right direction in the third embodiment.
FIG. 22A is a diagram illustrating an example of a composite image generated using a base color image having been subjected to the color depth cueing processing in which the light source is in a front direction in the third embodiment.
FIG. 22B is a diagram illustrating an example of a composite image generated using a base color image having not been subjected to the color depth cueing processing in which the light source is in the front direction in the third embodiment.
FIG. 23 is a block diagram illustrating an example of a configuration of a medical image processing apparatus according to a fourth embodiment.
FIG. 24 is a block diagram illustrating an example of a configuration of an ultrasonic diagnostic system according to a fifth embodiment.
FIG. 25 is a diagram illustrating an example of a configuration of an ultrasonic diagnostic apparatus according to the fifth embodiment.
FIG. 26 is a flowchart for describing an example of composite image generation processing executed in the ultrasonic diagnostic apparatus according to the fifth embodiment.
FIG. 27 is a diagram illustrating another example of the composite image generation processing executed in the ultrasonic diagnostic apparatus according to the fifth embodiment.

### DETAILED DESCRIPTION

Hereinafter, respective embodiments of the ultrasonic diagnostic apparatus, the medical image processing apparatus and the program will be described with reference to the accompanying drawings. In the embodiments below, the same reference signs are given for identical components in terms of configuration and function, and duplicate description is omitted.

### [First Embodiment]

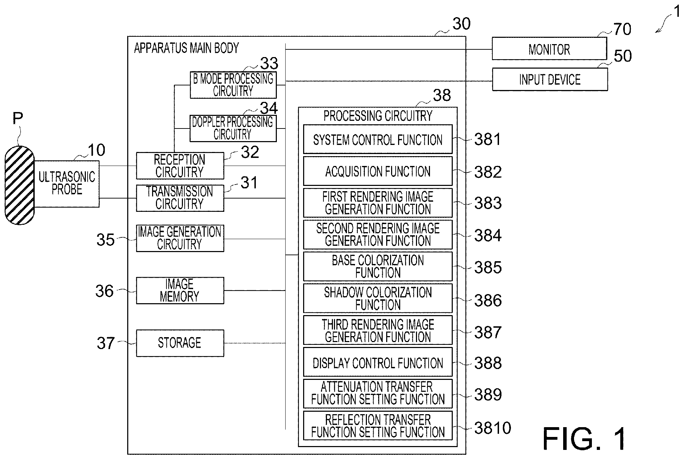

FIG. 1 is a block diagram illustrating an example of a configuration of an ultrasonic diagnostic apparatus according to a first embodiment. As illustrated in FIG. 1, an ultrasonic diagnostic apparatus 1 includes an ultrasonic probe 10, an apparatus main body 30, an input device 50, and a monitor 70.

The ultrasonic probe 10 includes, for example, a plurality of piezoelectric vibrators. The plurality of piezoelectric vibrators generate ultrasonic waves based on a drive signal supplied from transmission circuitry 31 included in the apparatus main body 30 to be described later. The ultrasonic probe 10 receives a reflected wave from a subject P and converts the reflected wave into an electrical signal. The ultrasonic probe 10 includes, for example, a matching layer provided on the piezoelectric vibrator, a backing material for preventing propagation of an ultrasonic wave rearward from the piezoelectric vibrator and the like.

When ultrasonic waves are transmitted from the ultrasonic probe 10 to the subject P, the transmitted ultrasonic waves are reflected one after another on a discontinuous surface of an acoustic impedance in a body tissue of the subject P, and are received as a reflected wave signal by the plurality of piezoelectric vibrators included in the ultrasonic probe 10. An amplitude of the received reflected wave signal depends on a difference in acoustic impedance at the discontinuous surface at which the ultrasonic waves are reflected. The reflected wave signal in a case where transmitted ultrasonic pulses are reflected by a moving blood flow or a surface of a heart wall or the like receives a frequency shift depending on a velocity component with respect to a transmission direction of an ultrasonic wave of a moving body, due to the Doppler effect.

The ultrasonic probe 10 is detachably connected to the apparatus main body 30. When scanning of a two-dimensional region in the subject P (two-dimensional scanning) is performed, the user connects, for example, a one-dimensional (1D) array probe in which a plurality of piezoelectric vibrators are arranged in a line to the apparatus main body 30 as the ultrasonic probe 10. The 1D array probe is a linear type ultrasonic probe, a convex type ultrasonic probe, a sector type ultrasonic probe, or the like. Further, when scanning of a three-dimensional region in the subject P (three-dimensional scanning) is performed, the user connects, for example, a mechanical four-dimensional (4D) probe or a two-dimensional (2D) array probe to the apparatus main body 30 as the ultrasonic probe 10. The mechanical 4D probe can perform two-dimensional scanning using a plurality of piezoelectric vibrators arranged in a line like the 1D array probe, and can perform three-dimensional scanning by swinging the plurality of piezoelectric vibrators at a predetermined angle (swing angle). The 2D array probe can perform three-dimensional scanning by a plurality of piezoelectric vibrators arranged in a matrix, and can perform two-dimensional scanning by focusing and transmitting ultrasonic waves. The 2D array probe can simultaneously perform two-dimensional scanning of a plurality of cross sections.

The apparatus main body 30 generates an ultrasonic image based on a signal from the ultrasonic probe 10. Specifically, the apparatus main body 30 can generate a two-dimensional ultrasonic image based on a reflected wave signal corresponding to a two-dimensional region of the subject P received by the ultrasonic probe 10. The apparatus main body 30 can generate a three-dimensional ultrasonic image based on a reflected wave signal corresponding to a three-dimensional region of the subject P received by the ultrasonic probe 10. As illustrated in FIG. 1, the apparatus main body 30 includes the transmission circuitry 31, reception circuitry 32, B mode processing circuitry 33, Doppler processing circuitry 34, image generation circuitry 35, an image memory 36, storage 37, and processing circuitry 38.

The transmission circuitry 31 includes a pulse generator, a transmission delay unit, a pulser, and the like, and supplies a drive signal to the ultrasonic probe 10. The pulse generator repeatedly generates rate pulses for forming transmission ultrasonic waves at a predetermined rate frequency. The transmission delay unit focuses ultrasonic waves generated from the ultrasonic probe 10 into a beam shape, and gives, to each rate pulse generated by the pulse generator, a delay time for each piezoelectric vibrator necessary to determine transmission directivity. Further, the pulser applies a drive signal (drive pulse) to the ultrasonic probe 10 at a timing based on the rate pulse. That is, the transmission delay unit arbitrarily adjusts the transmission direction of the ultrasonic wave transmitted from the piezoelectric vibrator surface by changing the delay time given to each rate pulse.

The transmission circuitry 31 has a function of instantaneously changing a transmission frequency, a transmission driving voltage, and the like in order to execute a predetermined scan sequence based on an instruction from the processing circuitry 38 to be described later. In particular, the transmission driving voltage is changed by linear amplifier type transmission circuitry capable of instantaneously switching the value thereof or a mechanism for electrically switching a plurality of power supply units.

The reception circuitry 32 includes a preamplifier, an analog/digital (A/D) converter, a reception delay unit, an adder, and the like, and performs various types of processing on the reflected wave signal received by the ultrasonic probe 10, thereby generating reflected wave data. The preamplifier amplifies the reflected wave signal for each channel and performs gain adjustment (gain correction). The A/D converter converts the gain-corrected reflected wave signal into a digital signal by A/D-converting the gain-corrected reflected wave signal. The reception delay unit gives a delay time necessary to determine reception directivity. The adder performs processing of adding reflected wave signals processed by the reception delay unit, and generates reflected wave data. Then, the adder outputs the generated reflected wave data to the B mode processing circuitry 33 or the Doppler processing circuitry 34.

When the subject P is two-dimensionally scanned, the transmission circuitry 31 causes the ultrasonic probe 10 to transmit a two-dimensional ultrasonic beam. Then, the reception circuitry 32 generates two-dimensional reflected wave data from a two-dimensional reflected wave signal received by the ultrasonic probe 10. Further, when the subject P is three-dimensionally scanned, the transmission circuitry 31 according to the present embodiment causes the ultrasonic probe 10 to transmit a three-dimensional ultrasonic beam. Then, the reception circuitry 32 generates three-dimensional reflected wave data from a three-dimensional reflected wave signal received by the ultrasonic probe 10.

The B mode processing circuitry 33 receives the reflected wave data from the reception circuitry 32, performs logarithmic amplification, envelope detection processing, and the like, and generates data (B mode data) in which signal intensity is expressed by brightness of luminance.

The Doppler processing circuitry 34 performs frequency analysis on velocity information from the reflected wave data received from the reception circuitry 32, extracts a blood flow, a tissue, and a contrast medium echo component by the Doppler effect, and generates data (Doppler data) obtained by extracting moving body information such as velocity, dispersion, and power for multiple points. Here, the moving body is, for example, a blood flow, a tissue of an organ that periodically moves such as a heart wall, or a contrast medium.

The B mode processing circuitry 33 and the Doppler processing circuitry 34 can process both types of two-dimensional reflected wave data and three-dimensional reflected wave data. That is, the B mode processing circuitry 33 generates two-dimensional B mode data from two-dimensional reflected wave data, and generates three-dimensional B mode data from three-dimensional reflected wave data. The Doppler processing circuitry 34 generates two-dimensional Doppler data from two-dimensional reflected wave data and generates three-dimensional Doppler data from three-dimensional reflected wave data. The B mode data and the Doppler data are also referred to as raw data.

The image generation circuitry 35 is a function of generating image data based on data generated by the B mode processing circuitry 33 and the Doppler processing circuitry 34. For example, the image generation circuitry 35 generates, as ultrasonic image data, two-dimensional B mode image data in which intensity of the reflected wave is represented by luminance from the two-dimensional B mode data generated by the B mode processing circuitry 33. The image generation circuitry 35 generates, as ultrasonic image data, two-dimensional Doppler image data as average speed image data, distributed image data, power image data, or a combination image thereof, representing the moving body information from the two-dimensional Doppler data generated by the Doppler processing circuitry 34.

For example, the image generation circuitry 35 generates M mode image data from time-series data of the B mode data on one scanning line generated by the B mode processing circuitry 33. The image generation circuitry 35 generates a Doppler waveform obtained by plotting a blood flow and tissue velocity information in time series from the Doppler data generated by the Doppler processing circuitry 34.

Furthermore, the image generation circuitry 35 can also generate three-dimensional B mode image data by performing coordinate conversion on the three-dimensional B mode data generated by the B mode processing circuitry 33. The image generation circuitry 35 can also generate three-dimensional Doppler image data by performing coordinate conversion on the three-dimensional Doppler data generated by the Doppler processing circuitry 34. That is, the image generation circuitry 35 generates "three-dimensional B mode image data and three-dimensional Doppler image data" as "three-dimensional ultrasonic data". In the following, the three-dimensional ultrasonic data is also referred to as ultrasonic volume data. The image generation circuitry 35 outputs the generated three-dimensional ultrasonic data to the image memory 36 and the processing circuitry 38. The image generation circuitry 35 is an example of an image generation unit.

The image memory 36 is a memory that stores various types of image data generated by the image generation circuitry 35, the processing circuitry 38, and the like. The image memory 36 also stores data generated by the B mode processing circuitry 33 and the Doppler processing circuitry 34. The B mode data and the Doppler data stored in the image memory 36 can be called by an operator after diagnosis, for example, and become display ultrasonic image data via the image generation circuitry 35 and the processing circuitry 38. The image memory 36 also stores the reflected wave data output from the reception circuitry 32. For example, the image memory 36 is implemented by a random access memory (RAM), a semiconductor memory element such as a flash memory, a hard disk, or an optical disk.

The storage 37 includes, for example, a storage medium readable by a processor, such as a magnetic storage medium, an optical storage medium, or a semiconductor memory. The storage 37 stores ultrasonic wave transmission and reception conditions, a program for implementing ultrasonic wave transmission and reception, a program related to global illumination rendering processing to be described later, a program related to surface rendering processing, a base color map for generating a base color image, a shadow color map for generating a shadow color image, various types of data, and the like. The storage 37 stores parameters used for photon mapping such as coordinate information used for photon mapping calculated in advance, a position and an orientation of a light source, and a clip plane and parameters used when generating a light attenuation image such as coordinate information calculated in advance, a position and an orientation of a light source, and a clip plane. The program and various types of data may be stored in advance in the storage 37, for example. The program and various types of data may be stored in a non-transitory storage medium and distributed, for example, and may be read from the non-transitory storage medium and installed in the storage 37.

The image memory 36 and the storage 37 are not necessarily implemented by independent storage devices. The image memory 36 and the storage 37 may be implemented by a single storage device. Each of the image memory 36 and the storage 37 may be implemented by a plurality of storage devices.

The processing circuitry 38 executes various types of data processing. The processing circuitry 38 is, for example, a processor that functions as a center of the ultrasonic diagnostic apparatus 1. In the following description, a case where the processing circuitry 38 is configured with a graphics processing unit (GPU) as a processor will be described. The GPU may store a texture having a plurality of channels. The plurality of channels included in the texture are, for example, four channels of RGBA. This texture is configured to be able to store image data based on three-dimensional ultrasonic data in each of the plurality of channels. In the present embodiment, in the R channel of the texture, a surface rendering image is stored by executing surface rendering processing. In the G channel of the texture, a global illumination rendering image is stored by executing photon mapping processing. The GPU of the processing circuitry 38 is an example of a texture memory.

The processing circuitry 38 includes a system control function 381, an acquisition function 382, a first rendering image generation function 383, a second rendering image generation function 384, a base colorization function 385, a shadow colorization function 386, a third rendering image generation function 387, a display control function 388, an attenuation transfer function setting function 389, and a reflection transfer function setting function 3810. Here, for example, the respective processing functions of the system control function 381, the acquisition function 382, the first rendering image generation function 383, the second rendering image generation function 384, the base colorization function 385, the shadow colorization function 386, the third rendering image generation function 387, the display control function 388, the attenuation transfer function setting function 389, and the reflection transfer function setting function 3810, which are components of the processing circuitry 38 illustrated in FIG. 1, are recorded in the storage 37 in the form of a program executable by a computer. The processing circuitry 38 reads each program from the storage 37 and executes each read program to implement a function corresponding to each program. In other words, the processing circuitry 38 in a state of reading each program has the functions of the processing circuitry 38 illustrated in FIG. 1. The processing circuitry 38 is implemented by, for example, a processor. The system control function 381 is an example of a system control unit. The acquisition function 382 is an example of an acquisition unit. The first rendering image generation function 383 is an example of a first acquisition unit. The second rendering image generation function 384 is an example of a second acquisition unit. The base colorization function 385 is an example of a color image generation unit. The shadow colorization function 386 is an example of the color image generation unit. The third rendering image generation function 387 is an example of a generation unit. The display control function 388 is an example of a display control unit. The attenuation transfer function setting function 389 is an example of an attenuation transfer function setting unit. The reflection transfer function setting function 3810 is an example of a reflection transfer function setting unit.

The system control function 381 is a function of integrally controlling the entire operation of the ultrasonic diagnostic apparatus 1. For example, the system control function 381 controls the transmission circuitry 31 and the reception circuitry 32 based on parameters related to transmission and reception conditions of ultrasonic waves according to various modes. Specifically, the system control function 381 acquires, from the storage 37 via the input device 50, the parameters related to transmission and reception conditions corresponding to a mode received from a user, and controls the transmission circuitry 31 and the reception circuitry 32 based on the parameters related to the transmission and reception conditions. The system control function 381 may receive various parameters such as the parameters related to transmission and reception conditions of ultrasonic waves according to various modes from the user via the input device 50. For this reason, the system control function 381 also functions as an input parameter processing function. The various modes include, for example, a B mode, a Doppler mode, and the like.

The acquisition function 382 is a function of acquiring three-dimensional ultrasonic data regarding a subject.

The first rendering image generation function 383 is a function of generating a surface rendering image by executing surface rendering processing on three-dimensional ultrasonic data. The first rendering image generation function 383 performs image processing (smoothing processing) of regenerating an average value image of luminance, image processing (edge enhancement processing) using a differential filter in an image, and the like. This surface rendering image is an example of a first image on which surface rendering has been executed on three-dimensional ultrasonic data related to a subject.

The second rendering image generation function 384 is a function of generating a light attenuation image, which is an image in which only light attenuation is recorded by executing global illumination rendering processing on the three-dimensional ultrasonic data. The light attenuation image is an example of a second image on which global illumination has been executed on the three-dimensional ultrasonic data.

The base colorization function 385 is a function of generating a base color image by applying a base color map to a surface rendering image. The base color map is an example of a first color map. The base color image is an example of a first color image.

The shadow colorization function 386 is a function of generating a shadow color image by applying a shadow color map different from the base color map to a surface rendering image. For example, the shadow color map is a darker color than the base color map. The shadow color map does not need to be a color darker than the base color map. That is, the shadow color map may be a lighter color than the base color map. The shadow color map is an example of a second color map. The shadow color image is an example of a second color image.

The third rendering image generation function 387 is a function of generating a composite image based on the surface rendering image and coefficients based on the light attenuation image. Specifically, the third rendering image generation function 387 generates a composite image by combining the base color image and the shadow color image using the coefficients based on the light attenuation image. The composite image is an example of a rendering image.

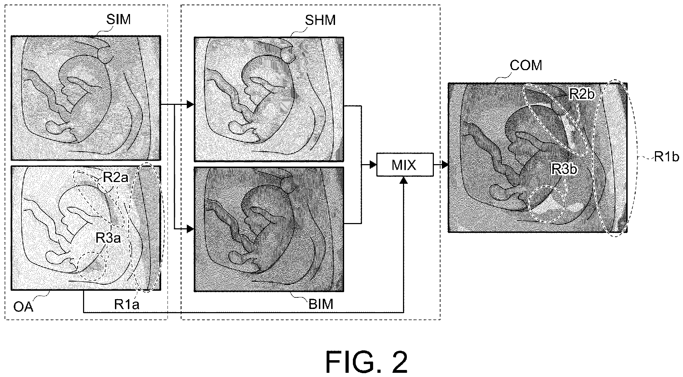

FIG. 2 is a diagram for describing a method of generating a composite image. In each image in the example illustrated in FIG. 2, since a viewpoint direction is a left direction, light from the light source travels in the same direction as the viewpoint direction. In each image illustrated in FIG. 2, a change in color tone is represented in grayscale. The whole body of a fetus is drawn in each image illustrated in FIG. 2.

As illustrated in FIG. 2, the first rendering image generation function 383 generates a surface rendering image SIM. The surface rendering image SIM is represented in grayscale. As illustrated in FIG. 2, the second rendering image generation function 384 generates a light attenuation image OA. The light attenuation image OA is also represented in grayscale. Then, as illustrated in FIG. 2, the base colorization function 385 generates a base color image BIM by applying an orange base color map to the surface rendering image SIM. As illustrated in FIG. 2, the shadow colorization function 386 generates a shadow color image SHM by applying a blue shadow color map to the surface rendering image SIM.

Then, the third rendering image generation function 387 generates a composite image COM by combining (MIX) the base color image BIM and the shadow color image SHM using coefficients based on the light attenuation image OA. Specifically, as illustrated in FIG. 2, a composite image COM1 is generated based on the base color image BIM by applying the shadow color image SHM1 to the region of the base color image BIM corresponding to regions R1a, R2a, and R3a of the light attenuation image OA in a portion having a small pixel value such as the regions R1a, R2a, and R3a in the light attenuation image OA, that is, a portion having large color attenuation. That is, as shown in the composite image COM of FIG. 2, regions R1b, R2b, and R3b of the composite image COM are configured with the shadow color image SHM, and regions other than the regions R1b, R2b, and R3b of the composite image COM are configured with the base color image BIM. Thereby, it is possible to add a depth feeling and a stereoscopic effect to the composite image COM1.

The display control function 388 is a function of controlling the monitor 70 so as to display the composite image. The display control function 388 displays, on the monitor 70, various frame lines, character information, and the like to be superimposed on the composite image.

The attenuation transfer function setting function 389 is a function of setting an attenuation transfer function for determining the degree of attenuation of light. The reflection transfer function setting function 3810 is a function of setting a reflection transfer function for determining the degree of reflection of light by determining luminance and/or color.

The input device 50 includes a mouse, a keyboard, a button, a panel switch, a touch command screen, a wheel, a dial, a foot switch, a trackball, a joystick, and the like. The input device 50 receives various setting requests and input operations for changing rendering parameters and transmission and reception conditions from the user of the ultrasonic diagnostic apparatus 1, and transfers the various setting requests and input operations received with respect to the apparatus main body 30 to the processing circuitry 38 and the like.

The monitor 70 displays a graphical user interface (GUI) for the user of the ultrasonic diagnostic apparatus 1 to input various setting requests using the input device 50, and displays an ultrasonic image, a Doppler waveform, and the like generated in the apparatus main body 30. The monitor 70 displays various messages in order to notify the user of a processing status of the apparatus main body 30. The monitor 70 includes a speaker and can also output sound. For example, the speaker of the monitor 70 outputs a predetermined sound such as a beep sound in order to notify the user of the processing status of the apparatus main body 30. The monitor 70 corresponds to a display unit according to the present embodiment.

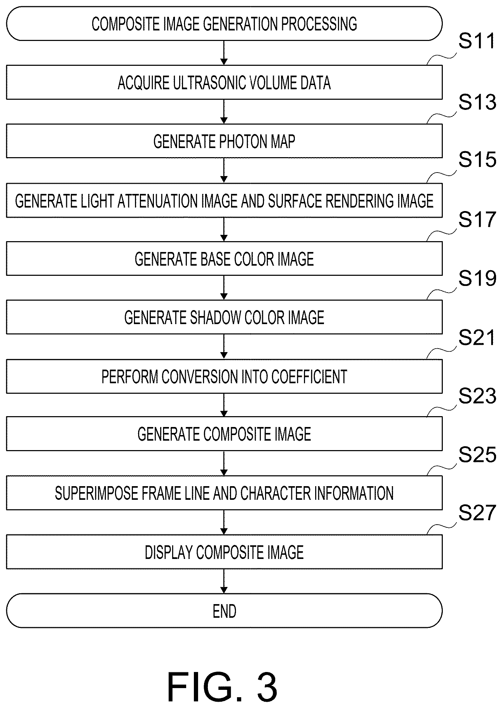

FIG. 3 is a flowchart for describing composite image generation processing executed in the ultrasonic diagnostic apparatus 1 according to the first embodiment. In the composite image generation processing, ultrasonic volume data is acquired, a photon map is generated, a light attenuation image and a surface rendering image are generated, a base color image is generated, a shadow color image is generated, a composite image is generated, a frame line and character information are superimposed, and a composite image is displayed. For example, the composite image generation processing is processing that is executed when the ultrasonic volume data is acquired.

As illustrated in FIG. 3, first, the acquisition function 382 in the processing circuitry 38 of the apparatus main body 30 acquires ultrasonic volume data (step S11). Specifically, the acquisition function 382 acquires the ultrasonic volume data from the image generation circuitry 35 or the image memory 36.

Next, as illustrated in FIG. 3, the second rendering image generation function 384 in the processing circuitry 38 of the apparatus main body 30 generates a photon map (step S13). Specifically, the second rendering image generation function 384 executes photon mapping processing as global illumination rendering processing on the ultrasonic volume data, and generates a photon map that is a map regarding attenuation of light generated by executing the photon mapping processing.

More specifically, the second rendering image generation function 384 performs various types of filter processing on the ultrasonic volume data and converts the ultrasonic volume data into voxel data of the Cartesian coordinate system, and then inputs the voxel data to the photon mapping processing. Then, in the photon mapping processing, the second rendering image generation function 384 generates a photon map using an attenuation transfer function that determines the degree of attenuation of light and is set for a different purpose for each of the RGBA channels. In the present embodiment, an attenuation transfer function is set for each of the R channel and the G channel among the RGBA channels. Everything is set to 0 and only a reflection component is set to be output for the attenuation transfer function of the R channel such that no light attenuation occurs, that is, no photon map is output from the R channel. A value to the extent that attenuation occurs is set for the attenuation transfer function of the G channel. The second rendering image generation function 384 executes the photon mapping processing on the ultrasonic volume data by using the attenuation transfer function and the parameters used for photon mapping, and generates a photon map.

Next, as illustrated in FIG. 3, the processing circuitry 38 of the apparatus main body 30 generates each of a surface rendering image and a light attenuation image (step S15). Specifically, the first rendering image generation function 383 generates a surface rendering image by executing surface rendering processing on three-dimensional ultrasonic data, and the second rendering image generation function 384 generates a light attenuation image based on the photon map generated by executing the photon mapping processing in step S13.

More specifically, the first rendering image generation function 383 generates a surface rendering image and the second rendering image generation function 384 generates a light attenuation image using a reflection transfer function for determining the degree of reflection of light by determining luminance and/or color set for a different purpose for each of the RGBA channels. For example, in the present embodiment, a reflection transfer function is set for each of the R channel and the G channel among the RGBA channels. The reflection transfer function of the R channel is set such that luminance and color generally used in surface rendering processing are monotonically increased, and the reflection transfer function of the G channel is set such that only the attenuation of light by the photon map is output as a light attenuation image, and all values are set to 255 as a uniform value.

Then, the first rendering image generation function 383 generates a surface rendering image by executing the surface rendering processing using the reflection transfer function of the R channel and the ultrasonic volume data. The second rendering image generation function 384 generates a light attenuation image based on the reflection transfer function of the G channel together with the photon map. Specifically, the second rendering image generation function 384 generates a light attenuation image by using the reflection transfer function of the G channel, the photon map, the parameters used when generating the light attenuation image, and the ultrasonic volume data. The surface rendering image is then stored in the R channel in the texture, and the light attenuation image is stored in the G channel in the texture. In this manner, when using a GPU, the surface rendering image and the global illumination rendering image are stored in independent channels and can be thus treated as a single texture. In the following, the surface rendering image is also referred to as a data buffer as necessary. In the following, the light attenuation image is also referred to as a shadow buffer as necessary.

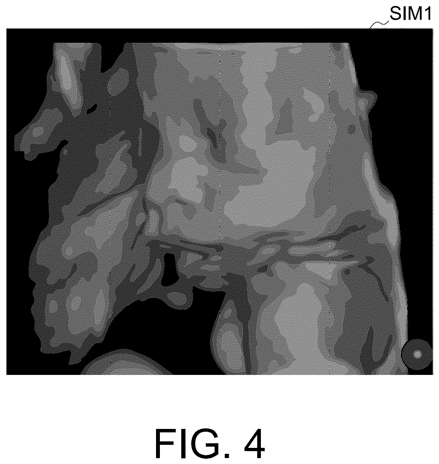

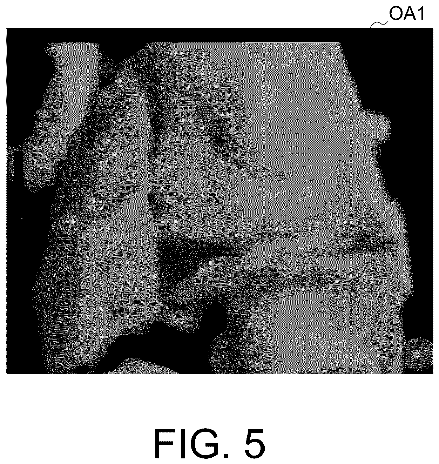

FIG. 4 is a diagram illustrating an example of a surface rendering image in the first embodiment. FIG. 5 is a diagram illustrating an example of a light attenuation image in the first embodiment. In the examples illustrated in FIGS. 4 and 5, since a viewpoint direction is a front direction, light from the light source travels in the same direction as the viewpoint direction. In a surface rendering image SIM1 illustrated in FIG. 4 and a light attenuation image OA1 illustrated in FIG. 5, a change in color tone is represented in grayscale. A fetus's face is drawn in each of the images illustrated in FIGS. 4 and 5.

Next, as illustrated in FIG. 3, the base colorization function 385 in the processing circuitry 38 of the apparatus main body 30 generates a base color image (step S17). Specifically, the base colorization function 385 generates a base color image by applying a base color map to the surface rendering image generated in step S15.

Next, as illustrated in FIG. 3, the shadow colorization function 386 in the processing circuitry 38 of the apparatus main body 30 generates a shadow color image (step S19). Specifically, the shadow colorization function 386 generates a shadow color image by applying a shadow color map to the surface rendering image generated in step S15.

Next, as illustrated in FIG. 3, the third rendering image generation function 387 in the processing circuitry 38 of the apparatus main body 30 converts the light attenuation image into a coefficient (step S21). Specifically, the third rendering image generation function 387 converts the light attenuation image into the coefficient by applying a coefficient conversion transfer function for converting a light attenuation image into a coefficient to the light attenuation image. The coefficient is, for example, a pixel value of the light attenuation image.

Next, as illustrated in FIG. 3, the third rendering image generation function 387 in the processing circuitry 38 of the apparatus main body 30 generates a composite image (step S23). Specifically, the third rendering image generation function 387 generates a composite image by combining the base color image generated in step S17 and the shadow color image generated in step S19 using the coefficient based on the light attenuation image converted in step S21. More specifically, the third rendering image generation function 387 combines the base color image and the shadow color image by linear interpolation by using the coefficient based on the light attenuation image. Thereby, the third rendering image generation function 387 generates a composite image. In the present embodiment, the third rendering image generation function 387 generates a composite image by linearly interpolating, with the shadow color image, a region of the base color image corresponding to a region where the pixel value in the coefficient based on the light attenuation image is close to 0 in the coefficient based on the light attenuation image.

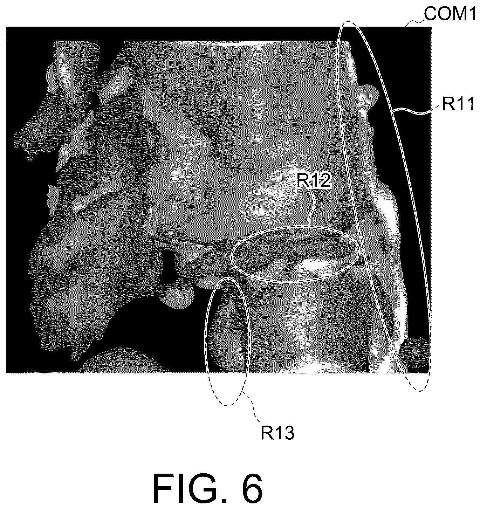

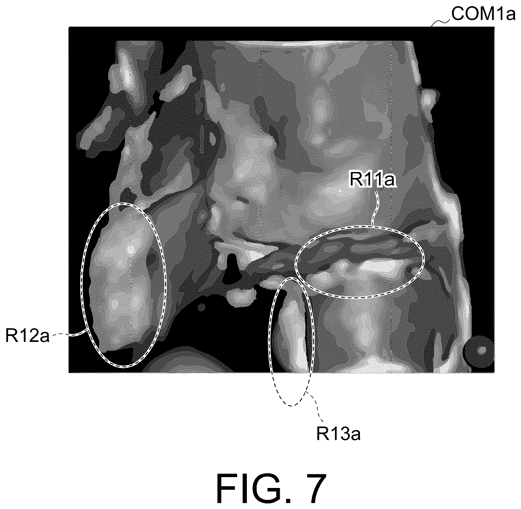

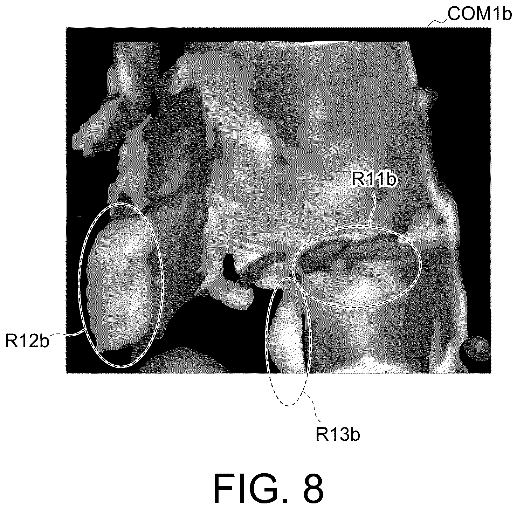

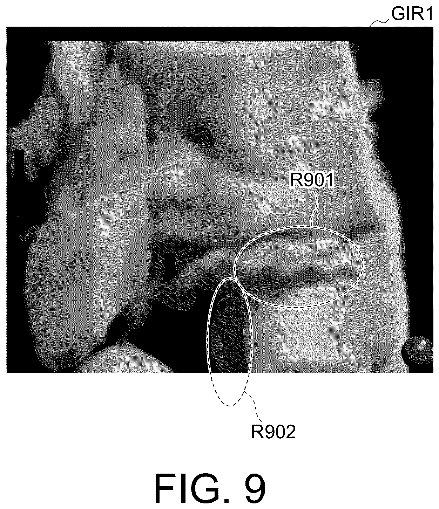

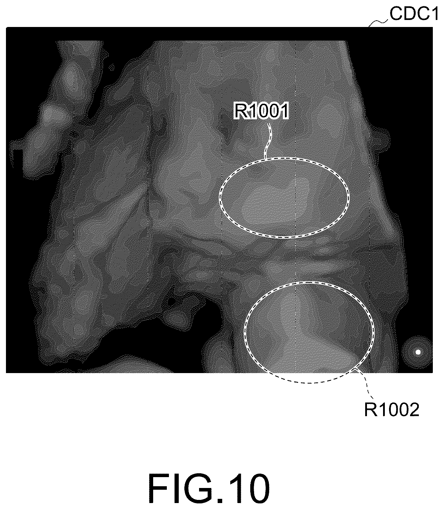

FIG. 6 is a diagram illustrating an example of a composite image in which a light source is in front in the first embodiment. FIG. 7 is a diagram illustrating an example of a composite image in which the light source is in a right direction in the first embodiment. FIG. 8 is a diagram illustrating an example of a composite image in which the light source is in an upper right direction in the first embodiment. Each of the composite images illustrated in FIGS. 6 to 8 is an image obtained by combining a surface rendering image to which a red base color map is applied and a surface rendering image to which a blue shadow color map is applied. Since the composite image COM1 illustrated in FIG. 6 is a composite image in which the light source is in front, a shadow color image is combined with a region R11 and a region R12, and thus it is possible to impart a depth feeling and a stereoscopic effect as a whole while imparting a shadow effect to the surface rendering image. Since a composite image COM1a illustrated in FIG. 7 is a composite image in which the light source is in the right direction, a shadow color image is combined with a region R11a, a region R12a, and a region R13a, and thus it is possible to impart a depth feeling and a stereoscopic effect as a whole while imparting a shadow effect to the surface rendering image. Since a composite image COM1b illustrated in FIG. 8 is a composite image in which the light source is in the upper right direction, a shadow color image is combined with a region R11b, a region R12b, and a region R13b, and thus it is possible to impart a depth feeling and a stereoscopic effect as a whole while imparting a shadow effect to the surface rendering image. On the other hand, in a global illumination rendering image by global illumination rendering processing of the related art or a color depth cueing image by color depth cueing processing of the related art, visibility is reduced or a stereoscopic effect is localized. In the following, an image using processing of the related art will be described with reference to FIGS. 9 and 10.

FIG. 9 is a diagram illustrating an example of a global illumination rendering image in which the light source is in the upper right direction, the global illumination rendering image being generated by global illumination rendering processing in the first embodiment. FIG. 10 is a diagram illustrating an example of a color depth cueing image generated by color depth cueing processing in the first embodiment. A fetus's face is drawn in each of the global illumination rendering image and the color depth cueing image illustrated in FIGS. 9 and 10, similarly to FIGS. 6 to 8. A global illumination rendering image GIR1 in FIG. 9 has a depth feeling and a stereoscopic effect by imparting a shadow to a region R901 and the like, but since light does not hit a shadow portion as in a region R902, visibility is reduced. In the global illumination rendering processing, ambient light can be applied to the shadow portion, but the calculation cost increases. A color depth cueing image CDC1 in FIG. 10 has a depth feeling due to changes in color tone of a region R1001 and a region R1002, but since no shadow effect is imparted, a stereoscopic effect is localized.

Next, as illustrated in FIG. 3, the display control function 388 superimposes a frame line and character information (step S25). Specifically, the display control function 388 superimposes the frame line and the character information on the composite image generated in step S23. Thereby, a display image to be displayed on the monitor 70 is generated.

Next, as illustrated in FIG. 3, the display control function 388 displays the composite image (step S27). Specifically, the display control function 388 controls the monitor 70 to display the composite image in which the frame line and the character information are superimposed in step S25.

In step S27, the composite image generation processing ends by displaying the composite image.

As described above, in the ultrasonic diagnostic apparatus 1, ultrasonic volume data is acquired, surface rendering processing is executed on the acquired ultrasonic volume data to generate a surface rendering image, global illumination rendering processing is executed on the ultrasonic volume data to generate a light attenuation image, and a composite image is generated based on the surface rendering image and a coefficient based on the light attenuation image. Thus, it is possible to generate a rendering image capable of securing visibility of a shadow portion while imparting a shadow effect.

In the ultrasonic diagnostic apparatus 1, since a light attenuation image (shadow buffer) is an image for obtaining a coefficient used for combining a base color image and a shadow color image, the light attenuation image does not need to have high resolution, and even when the light attenuation image has low resolution, resolution of a composite image is not affected. For this reason, it is possible to reduce a calculation amount of photon mapping processing executed as global illumination rendering processing in the ultrasonic diagnostic apparatus 1 by lowering the resolution of the light attenuation image.

In the ultrasonic diagnostic apparatus 1, a light attenuation image and a surface rendering image can be simultaneously generated, and thus a calculation amount of the processing circuitry 38 can be reduced.

### [Second Embodiment]

In the first embodiment described above, the ultrasonic diagnostic apparatus 1 simultaneously generates a light attenuation image and a surface rendering image, but the light attenuation image and the surface rendering image may be generated separately. In the following, differences from the first embodiment described above will be described.

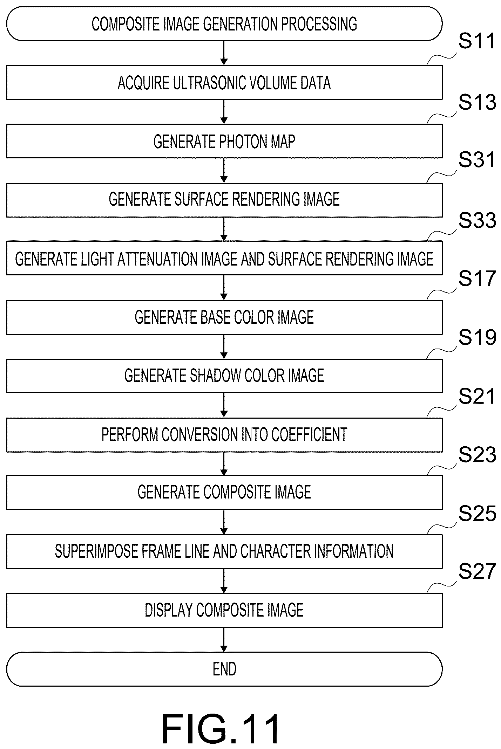

FIG. 11 is a flowchart for describing the content of composite image generation processing executed in an ultrasonic diagnostic apparatus 1 according to a second embodiment, and is a diagram corresponding to FIG. 3. In the composite image generation processing, ultrasonic volume data is acquired, a photon map is generated, a light attenuation image is generated, a surface rendering image is generated, a base color image is generated, a shadow color image is generated, a composite image is generated, a frame line and character information are superimposed, and a composite image is displayed. For example, the composite image generation processing is processing that is executed when the ultrasonic volume data is acquired. Processing of steps S11 and S13 in the composite image generation processing illustrated in FIG. 11 is the same as that in FIG. 3, and thus the description thereof is omitted.

Next, as illustrated in FIG. 11, the second rendering image generation function 384 in the processing circuitry 38 of the apparatus main body 30 generates a light attenuation image (step S31). Specifically, the second rendering image generation function 384 generates a light attenuation image based on the photon map generated by executing the photon mapping processing in step S13. More specifically, the second rendering image generation function 384 generates a light attenuation image by using a reflection transfer function in which all values are set to 255 as a uniform value such that only the attenuation of light by the photon map is output as a light attenuation image, parameters used when generating the light attenuation image such as coordinate information, a position and an orientation of a light source, and a clip plane, and the photon map.

Next, as illustrated in FIG. 11, the first rendering image generation function 383 in the processing circuitry 38 of the apparatus main body 30 generates a surface rendering image (step S33). Specifically, the first rendering image generation function 383 generates a surface rendering image by executing surface rendering processing on three-dimensional ultrasonic data. More specifically, in the surface rendering processing, the first rendering image generation function 383 performs filtering processing on ultrasonic volume data, and then generates a surface rendering image using the reflection transfer function that is set such that generally used luminance and color are monotonically increased.

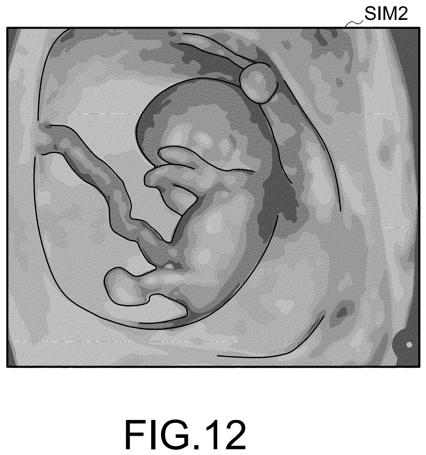

FIG. 12 is a diagram illustrating an example of a surface rendering image according to the second embodiment, and is a diagram corresponding to FIG. 4. In a surface rendering image SIM2 illustrated in FIG. 12, a change in color tone is represented in grayscale. The whole body of a fetus is drawn in the surface rendering image SIM2 illustrated in FIG. 12.

Processing of step S17 and the subsequent steps after step S33 is the same as that in FIG. 3, and thus the description thereof is omitted. In step S27, the composite image generation processing ends by displaying the composite image.

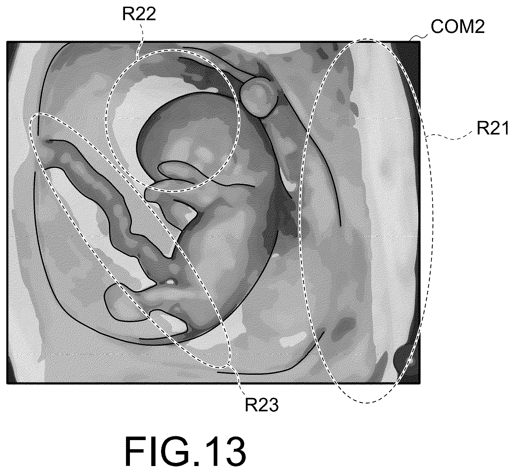

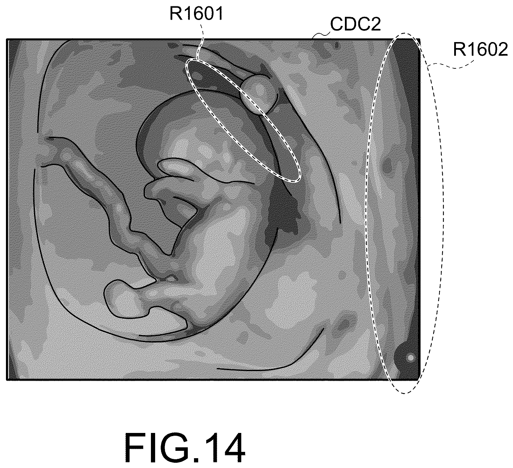

FIG. 13 is a diagram illustrating an example of a composite image in which a light source is in front in the second embodiment, and is a diagram corresponding to FIG. 6. Each of the composite images illustrated in FIG. 13 is an image obtained by combining a surface rendering image to which an orange base color map is applied and a surface rendering image to which a blue shadow color map is applied. Since a composite image COM2 illustrated in FIG. 13 is a composite image when the light source is in front, a shadow color image is combined with a region R21, a region R22, and a region R23, and thus it is possible to impart a depth feeling and stereoscopic effect as a whole while imparting a shadow effect to the surface rendering image. On the other hand, in a global illumination rendering image by global illumination rendering processing of the related art or a color depth cueing image by color depth cueing processing of the related art, visibility is reduced or a stereoscopic effect is localized. In the following, as processing of the related art, a color depth cueing image using color depth cueing processing will be described with reference to FIG. 14.

FIG. 14 is a diagram illustrating an example of a color depth cueing image generated by color depth cueing processing in the second embodiment. In a color depth cueing image CDC2 illustrated in FIG. 14, the whole body of a fetus is drawn as in FIG. 13. The color depth cueing image CDC2 in FIG. 14 has a depth feeling due to changes in color tone of a region R1601 and a region R1602, but since no shadow effect is imparted, a stereoscopic effect is localized.

As described above, also in the ultrasonic diagnostic apparatus 1 according to the second embodiment, ultrasonic volume data is acquired, surface rendering processing is executed on the acquired ultrasonic volume data to generate a surface rendering image, global illumination rendering processing is executed on the ultrasonic volume data to generate a light attenuation image, and a composite image is generated based on the surface rendering image and a coefficient based on the light attenuation image. Thus, it is possible to generate a rendering image capable of securing visibility of a shadow portion while imparting a shadow effect.

Also in the ultrasonic diagnostic apparatus 1 according to the second embodiment, since a light attenuation image (shadow buffer) is an image for obtaining a coefficient used for combining a base color image and a shadow color image, the light attenuation image does not need to have high resolution, and even when the light attenuation image has low resolution, resolution of a composite image is not affected. For this reason, it is possible to reduce a calculation amount of photon mapping processing executed as global illumination rendering processing in the ultrasonic diagnostic apparatus 1 by lowering the resolution of the light attenuation image.

In the ultrasonic diagnostic apparatus 1 according to the second embodiment, since a light attenuation image and a surface rendering image are separately generated, input of ultrasonic volume data can be set separately, and each of the light attenuation image and the surface rendering image can be generated with optimum settings.

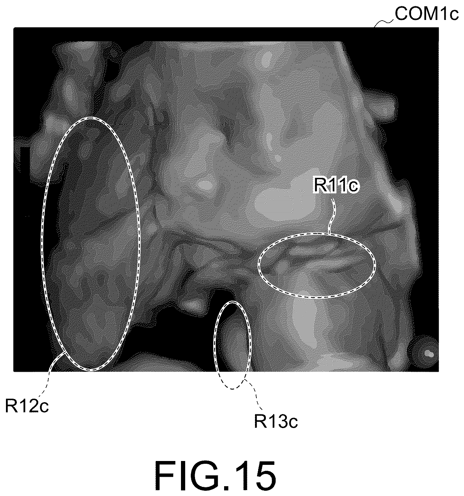

In the ultrasonic diagnostic apparatuses 1 according to the first and second embodiments described above, the orange base color map is used as the base color map, and the blue color map is used as the shadow color map. However, a combination of colors of the base color map and the shadow color map is not limited thereto. That is, a combination of colors of the base color map and the shadow color map is arbitrary, and may be, for example, a combination of an orange base color map and a deep blue shadow color map. FIG. 15 is a diagram illustrating an example of a composite image in which the light source is in a right direction, and is a diagram corresponding to FIG. 7. A composite image COM1c illustrated in FIG. 15 is an image obtained by combining a surface rendering image to which an orange base color map is applied and a surface rendering image to which a deep blue shadow color map is applied. Since the composite image COM1c illustrated in FIG. 15 is a composite image in which the light source is in the right direction, a shadow color image is combined with a region R11c, a region R12c, and a region R13c, and thus it is possible to impart a depth feeling and stereoscopic effect as a whole while imparting a shadow effect to the surface rendering image. As illustrated in FIG. 15, the composite image COM1c can be given a shadow effect without reducing visibility as shown in the region R13c.

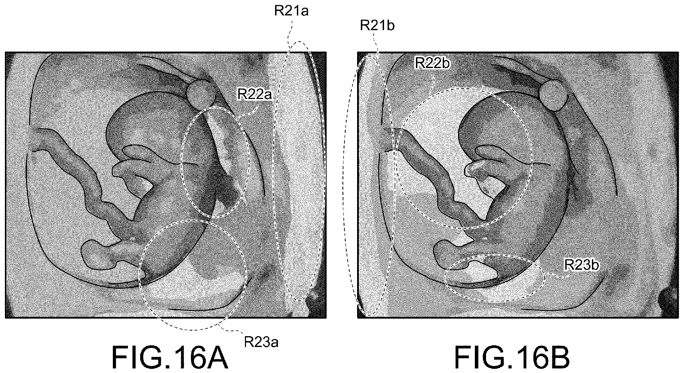

In step S27, after displaying the composite image, the user can adjust the composite image by moving the position of the light source, and the like. FIG. 16A is a diagram illustrating an example of a composite image in which the light source is on an upper left side in the second embodiment, and FIG. 16B is a diagram illustrating an example of a composite image in which the light source is on an upper right side in the second embodiment. In the example illustrated in FIG. 16A, the shadow color image is combined with the position of each of a region R21a, a region R22a, and a region R23a. On the other hand, in the example illustrated in FIG. 16B, the shadow color image is combined with the position of each of a region R21b, a region R22b, and a region R23b by moving the position of the light source from the upper left side to the upper right side. That is, as illustrated in FIGS. 16A and 16B, the user can adjust the composite image by moving the position of the light source.

### [Third Embodiment]

In the ultrasonic diagnostic apparatuses 1 according to the first and second embodiments described above, color depth cueing processing may be executed. A case in which this modification is applied to the first embodiment will be referred to as a third embodiment, and differences from the above-described first embodiment will be described. This modification can also be applied to the second embodiment.

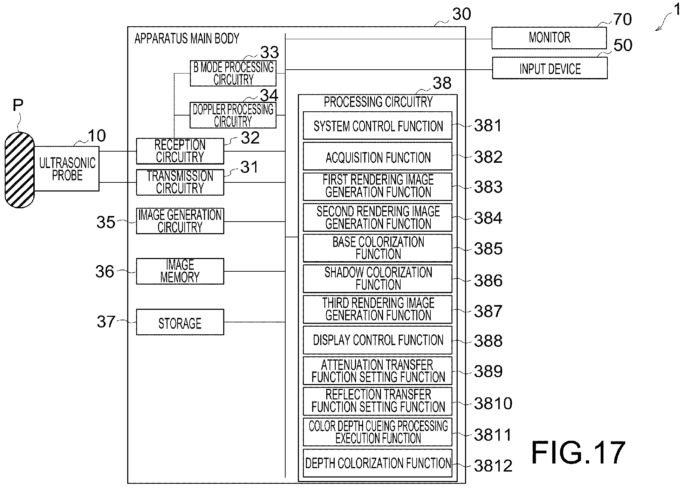

FIG. 17 is a block diagram illustrating an example of a configuration of an ultrasonic diagnostic apparatus 1 according to the third embodiment, and is a diagram corresponding to FIG. 1. As illustrated in FIG. 17, the ultrasonic diagnostic apparatus according to the present embodiment is configured by adding a color depth cueing processing execution function 3811 and a depth colorization function 3812 to the processing circuitry 38 as compared with the ultrasonic diagnostic apparatus 1 according to the first embodiment. Configurations and functions other than the color depth cueing processing execution function 3811 and the depth colorization function 3812 are the same as those in FIG. 1, and thus the description thereof is omitted.

The color depth cueing processing execution function 3811 is a function of executing color depth cueing processing on a surface rendering image. Here, the color depth cueing processing is processing for changing a color in accordance with a distance from a light source, such as brightening a portion close to the light source and darkening a portion far from the light source. The color depth cueing processing execution function 3811 is an example of a first processing execution unit.

The depth colorization function 3812 is a function of generating a depth color image by applying a depth color map different from the base color map and the shadow color map to the surface rendering image. The depth color map is an example of a third color map. The depth color image is an example of a third color image.

In the present embodiment, in an R channel of a texture stored in the processing circuitry 38, a surface rendering image is stored by executing surface rendering processing. In a G channel of the texture, a global illumination rendering image is stored by executing photon mapping processing, and in a B channel of the texture, a depth image to be described later is stored. Thereby, the surface rendering image, the global illumination rendering image, and the depth image can be treated as a single texture.

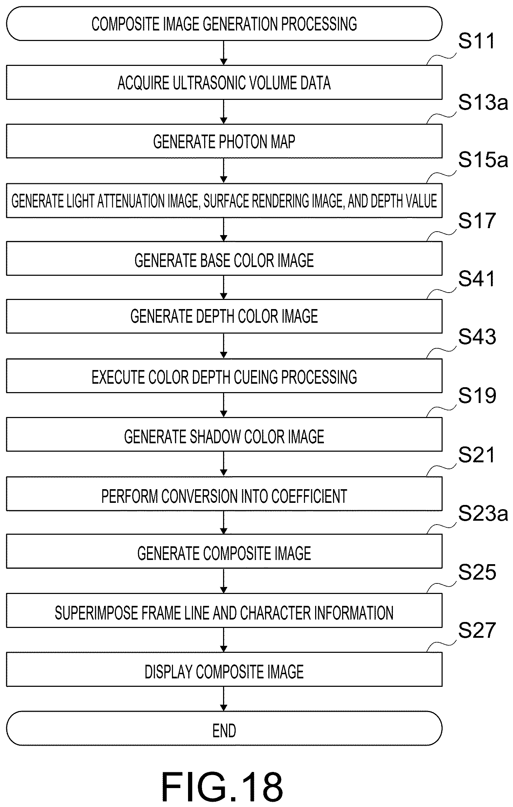

FIG. 18 is a flowchart for describing composite image generation processing executed in the ultrasonic diagnostic apparatus 1 according to the third embodiment, and is a diagram corresponding to FIG. 3. In the composite image generation processing, ultrasonic volume data is acquired, a photon map is generated, a light attenuation image, a surface rendering image, and a depth image are generated, a base color image is generated, a depth color image is generated, color depth cueing processing is executed, a shadow color image is generated, a composite image is generated, a frame line or character information is superimposed, and a composite image is displayed. For example, the composite image generation processing is processing that is executed when the ultrasonic volume data is acquired. Processing of step S11 is the same as that in FIG. 3, and thus the description thereof will be omitted.

Next, as illustrated in FIG. 18, the second rendering image generation function 384 in the processing circuitry 38 of the apparatus main body 30 generates a photon map (step S13a). Specifically, the second rendering image generation function 384 executes photon mapping processing as global illumination rendering processing on the ultrasonic volume data, and generates a photon map that is a map regarding attenuation of light generated by executing the photon mapping processing.

More specifically, the second rendering image generation function 384 performs various types of filter processing on the ultrasonic volume data and converts the ultrasonic volume data into voxel data of the Cartesian coordinate system, and then inputs the voxel data to the photon mapping processing. Then, in the third embodiment, in the photon mapping processing, the second rendering image generation function 384 generates a photon map using an attenuation transfer function that determines the degree of attenuation of light and is set for a different purpose for each of the RGBA channels. In the present embodiment, an attenuation transfer function is set for each of the R channel, the G channel, and the B channel among the RGBA channels. Everything is set to 0 and only a reflection component is set to be output for the attenuation transfer function of the R channel such that no light attenuation occurs, that is, no photon map is output from the R channel. A value to the extent that attenuation occurs is set for the attenuation transfer function of the G channel. Similarly to the attenuation transfer function of the R channel, everything is set to 0 for the attenuation transfer function of the B channel such that no light attenuation occurs. The second rendering image generation function 384 executes the photon mapping processing on the ultrasonic volume data by using the attenuation transfer function and the parameters used for photon mapping, and generates a photon map.

Next, as illustrated in FIG. 18, the processing circuitry 38 of the apparatus main body 30 generates each of a surface rendering image, a light attenuation image, and a depth image (step S15a). Specifically, the first rendering image generation function 383 generates a surface rendering image by executing surface rendering processing on three-dimensional ultrasonic data, the second rendering image generation function 384 generates a light attenuation image based on the photon map generated by executing the photon mapping processing in step S13, and the color depth cueing processing execution function 3811 generates a depth image based on the three-dimensional ultrasonic data.

More specifically, by using a reflection transfer function for determining the degree of reflection of light by determining luminance and/or color set for a different purpose for each of the RGBA channels, the first rendering image generation function 383 generates a surface rendering image, the second rendering image generation function 384 generates a light attenuation image, and the color depth cueing processing execution function 3811 generates a depth image. For example, in the present embodiment, a reflection transfer function is set for each of the R channel, the G channel, and the B channel among the RGBA channels. For the reflection transfer function of the R channel, a reflection transfer function is set such that luminance and color generally used in surface rendering processing are monotonically increased, the reflection transfer function of the G channel is set such that only the attenuation of light by the photon map is output as a light attenuation image and all values are set to 255 as a uniform value, and the reflection transfer function of the B channel is set such that luminance and color are monotonically increased or monotonically decreased while maintaining a positional relationship of depth.

Then, the first rendering image generation function 383 generates a surface rendering image by executing the surface rendering processing using the reflection transfer function of the R channel and the ultrasonic volume data. The second rendering image generation function 384 generates a light attenuation image based on the reflection transfer function of the G channel together with the photon map. Specifically, the second rendering image generation function 384 generates a light attenuation image by using the reflection transfer function of the G channel, the photon map, the parameters used when generating the light attenuation image, and the ultrasonic volume data. The color depth cueing processing execution function 3811 calculates three-dimensional depth information based on a position and an orientation of a light source by inputting the ultrasonic volume data acquired in step S11 to the B channel, and generates a two-dimensional depth image having brightness corresponding to the depth information. The surface rendering image is then stored in the R channel in the texture, and the light attenuation image is stored in the G channel in the texture, and the two-dimensional depth image is stored in the B channel. In this manner, when using a GPU, the surface rendering image, the global illumination rendering image, and the depth image are stored in independent channels and can be thus treated as a single texture. In the following, the surface rendering image is also referred to as a data buffer as necessary. In the following, the light attenuation image is also referred to as a shadow buffer as necessary. In the following, the depth image is also referred to as a depth buffer or a Z buffer as necessary. Processing of step S17 is the same as that in FIG. 3, and thus the description thereof will be omitted.

Next, as illustrated in FIG. 18, the depth colorization function 3812 in the processing circuitry 38 of the apparatus main body 30 generates a depth color image (step S41). Specifically, the depth colorization function 3812 generates a depth color image by applying the depth color map to the surface rendering image generated in step S15.

Next, as illustrated in FIG. 18, the color depth cueing processing execution function 3811 in the processing circuitry 38 of the apparatus main body 30 executes color depth cueing processing (step S43). Specifically, by executing the color depth cueing processing, the color depth cueing processing execution function 3811 combines the base color image generated in step S17 and the depth color image generated in step S41 using the depth buffer as a coefficient to generate a base color image having been subjected to the color depth cueing processing. More specifically, by executing the color depth cueing processing, the color depth cueing processing execution function 3811 combines the base color image generated in step S17 and the depth color image generated in step S41 by linear interpolation by using the depth buffer as a coefficient. Thereby, the color depth cueing processing execution function 3811 generates the base color image having been subjected to the color depth cueing processing. Processing of step S19 and step S21 after step S43 is the same as that in FIG. 3, and thus the description thereof will be omitted.

Next, as illustrated in FIG. 18, the third rendering image generation function 387 in the processing circuitry 38 of the apparatus main body 30 generates a composite image (step S23a). Specifically, the third rendering image generation function 387 generates a composite image by combining the base color image having been subjected to the color depth cueing processing generated in step S43 and the shadow color image generated in step S19 using the coefficient based on the light attenuation image converted in step S21. More specifically, the third rendering image generation function 387 combines the base color image having been subjected to the color depth cueing processing and the shadow color image by linear interpolation by using the coefficient based on the light attenuation image. Thereby, the third rendering image generation function 387 generates a composite image. In the present embodiment, the third rendering image generation function 387 generates a composite image by linearly interpolating, with the shadow color image, a region of the base color image having been subjected to the color depth cueing processing corresponding to a region where the pixel value in the coefficient based on the light attenuation image is close to 0 in the coefficient based on the light attenuation image.

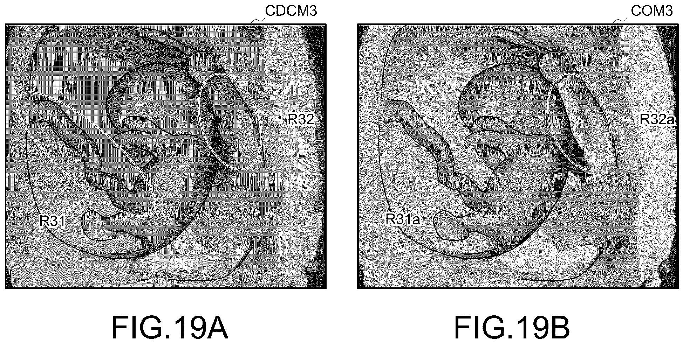

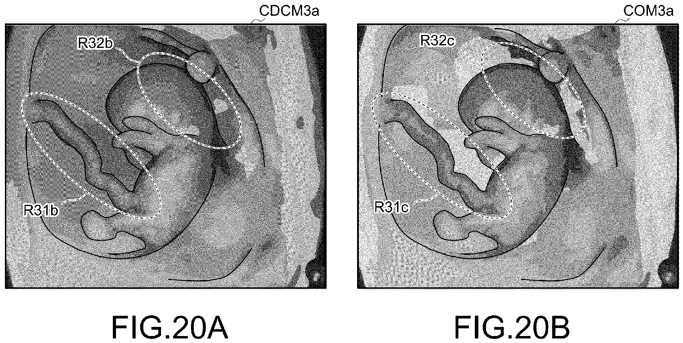

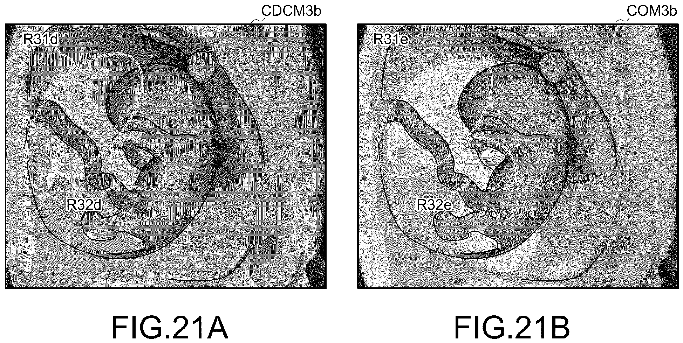

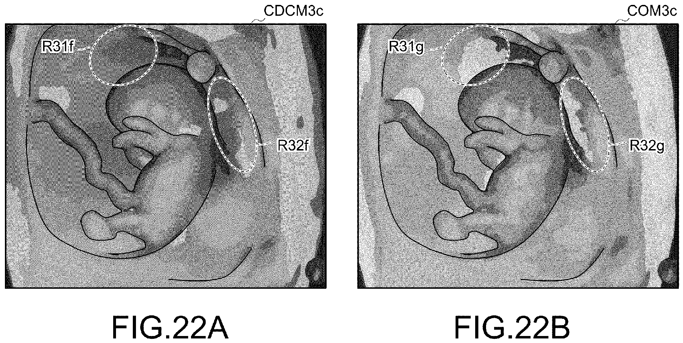

With reference to FIGS. 19A to 21B, a comparison between a composite image generated using a base color image having been subjected to the color depth cueing processing and a composite image generated using a base color image having not been subjected to the color depth cueing processing will be described. FIG. 19A is a diagram illustrating an example of a composite image generated using a base color image having been subjected to the color depth cueing processing in which the light source is in an upper left direction in the third embodiment, and FIG. 19B is a diagram illustrating an example of a composite image generated using a base color image having not been subjected to the color depth cueing processing in which the light source is in the upper left direction in the third embodiment. FIG. 20A is a diagram illustrating an example of a composite image generated using a base color image having been subjected to the color depth cueing processing in which the light source is in a lower left direction in the third embodiment, and FIG. 20B is a diagram illustrating an example of a composite image generated using a base color image having not been subjected to the color depth cueing processing in which the light source is in the lower left direction in the third embodiment. FIG. 21A is a diagram illustrating an example of a composite image generated using a base color image having been subjected to the color depth cueing processing in which the light source is in an upper right direction in the third embodiment, and FIG. 21B is a diagram illustrating an example of a composite image generated using a base color image having not been subjected to the color depth cueing processing in which the light source is in the upper right direction in the third embodiment. FIG. 22A is a diagram illustrating an example of a composite image generated using a base color image having been subjected to the color depth cueing processing in which the light source is in a front direction in the third embodiment, and FIG. 22B is a diagram illustrating an example of a composite image generated using a base color image having not been subjected to the color depth cueing processing in which the light source is in the front direction in the third embodiment. In each of the images illustrated in FIGS. 19A to 22B, the whole body of a fetus is drawn, similarly to the second embodiment described above.

Since a composite image COM3 generated using the base color image having not been subjected to the color depth cueing processing illustrated in FIG. 19B is a composite image in which the light source is on the upper left side, it is possible to impart a depth feeling and a stereoscopic effect as a whole while imparting a shadow effect to the surface rendering image by combining a shadow color image with a region R31a and a region R32a. On the other hand, in a composite image CDCM3 generated using the base color image having been subjected to the color depth cueing processing illustrated in FIG. 19A, a shadow color image is combined with a region R31 and a region R32 corresponding to the region R31a and the region R32a, and thus it is possible to impart a depth feeling and a stereoscopic effect by the color depth cueing processing while imparting a shadow effect to the surface rendering image and imparting a depth feeling and a stereoscopic effect as a whole.

Similarly to the cases of FIGS. 19A and 19B, since a composite image COM3a generated using the base color image having not been subjected to the color depth cueing processing illustrated in FIG. 20B is a composite image in which the light source is on the lower left side, it is possible to impart a depth feeling and a stereoscopic effect as a whole while imparting a shadow effect to the surface rendering image by combining a shadow color image with a region R31c and a region R32c. On the other hand, in a composite image CDCM3a generated using the base color image having been subjected to the color depth cueing processing illustrated in FIG. 20A, a shadow color image is combined with a region R31b and a region R32b corresponding to the region R31c and the region R32c, and thus it is possible to impart a depth feeling and a stereoscopic effect by the color depth cueing processing while imparting a shadow effect to the surface rendering image and imparting a depth feeling and a stereoscopic effect as a whole.

Similarly to the cases of FIGS. 19A to 20B, since a composite image COM3b generated using the base color image having not been subjected to the color depth cueing processing illustrated in FIG. 21B is a composite image in which the light source is on the lower left side, it is possible to impart a depth feeling and a stereoscopic effect as a whole while imparting a shadow effect to the surface rendering image by combining a shadow color image with a region R31e and a region R32e. On the other hand, in a composite image CDCM3b generated using the base color image having been subjected to the color depth cueing processing illustrated in FIG. 21A, a shadow color image is combined with a region R31d and a region R32d corresponding to the region R31e and the region R32e, and thus it is possible to impart a depth feeling and a stereoscopic effect by the color depth cueing processing while imparting a shadow effect to the surface rendering image and imparting a depth feeling and a stereoscopic effect as a whole.

Further, similarly to the cases of FIGS. 19A to 21B, since a composite image COM3c generated using the base color image having not been subjected to the color depth cueing processing illustrated in FIG. 22B is a composite image in which the light source is on the lower left side, it is possible to impart a depth feeling and a stereoscopic effect as a whole while imparting a shadow effect to the surface rendering image by combining a shadow color image with a region R31g and a region R32g. On the other hand, in a composite image CDCM3b generated using the base color image having been subjected to the color depth cueing processing illustrated in FIG. 22A, a shadow color image is combined with a region R31f and a region R32f corresponding to the region R31g and the region R32g, and thus it is possible to impart a depth feeling and a stereoscopic effect by the color depth cueing processing while imparting a shadow effect to the surface rendering image and imparting a depth feeling and a stereoscopic effect as a whole.

Processing of step S25 and the subsequent steps after step S23a is the same as that in FIG. 3, and thus the description thereof is omitted. In step S27, the composite image generation processing ends by displaying the composite image.

As described above, also in the ultrasonic diagnostic apparatus 1 according to the third embodiment, ultrasonic volume data is acquired, surface rendering processing is executed on the acquired ultrasonic volume data to generate a surface rendering image, global illumination rendering processing is executed on the ultrasonic volume data to generate a light attenuation image, and a composite image is generated based on the surface rendering image having been subjected to the color depth cueing processing and a coefficient based on the light attenuation image. Thus, it is possible to generate a rendering image capable of securing visibility of a shadow portion while imparting a shadow effect.

In the ultrasonic diagnostic apparatus 1 according to the third embodiment described above, color depth cueing processing is executed before generating a composite image, but the order of processing for generating the composite image and processing for executing the color depth cueing processing is not limited thereto. That is, the order of the processing for generating the composite image and the processing for executing the color depth cueing processing is arbitrary, and the color depth cueing processing may be executed on the composite image after the processing for generating the composite image is executed.

### [Fourth Embodiment]

In the first to third embodiments, a case where the ultrasonic diagnostic apparatus 1 has a plurality of functions has been described, but the plurality of functions may be included in a medical image processing apparatus. In the following, the present embodiment will be referred to as a fourth embodiment, and differences from the first to third embodiments will be described.

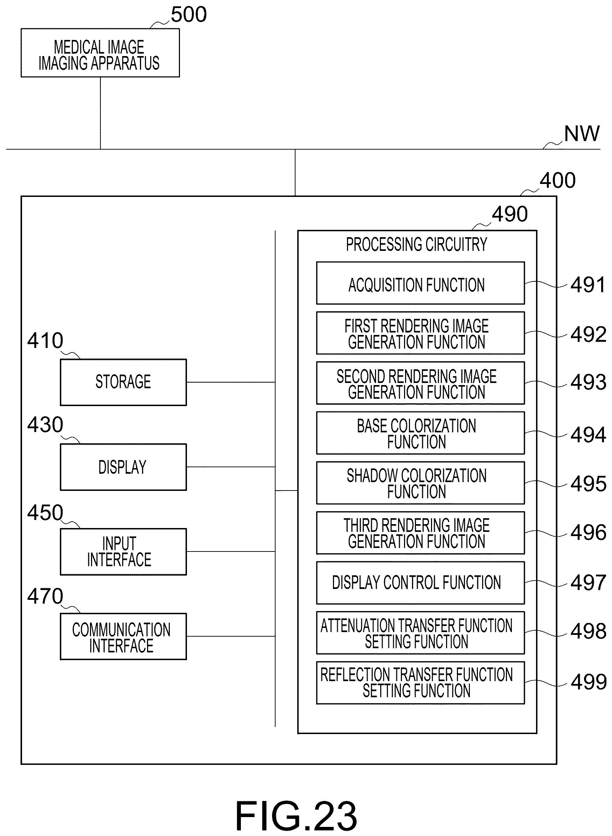

FIG. 23 is a block diagram illustrating an example of a configuration of a medical image processing apparatus according to the fourth embodiment. As illustrated in FIG. 23, a medical image processing apparatus 400 is connected to a medical image capturing apparatus 500 via an in-hospital network NW. The medical image capturing apparatus 500 corresponds to, for example, an ultrasonic diagnostic apparatus. As illustrated in FIG. 23, the medical image processing apparatus 400 includes storage 410, a display 430, an input interface 450, a communication interface 470, and processing circuitry 490.

The storage 410 includes, for example, a storage medium readable by a processor, such as a magnetic storage medium, an optical storage medium, or a semiconductor memory. The storage 410 stores ultrasonic wave transmission and reception conditions, a program for implementing ultrasonic wave transmission and reception, a program related to global illumination rendering processing to be described later, a program related to surface rendering processing, a base color map for generating a base color image, a shadow color map for generating a shadow color image, various types of data, and the like. The program and various types of data may be stored in advance in the storage 410, for example. The program and various types of data may be stored in a non-transitory storage medium and distributed, for example, and may be read from the non-transitory storage medium and installed in the storage 410. The storage 410 stores various types of image data generated by the medical image capturing apparatus 500.

The storage 410 may be a drive device or the like that reads and writes various types of information from and to a portable storage medium such as a CD drive, a DVD drive, and a flash memory. The storage 410 can also write stored data to the portable storage medium and store the data in an external device via the portable storage medium.

The display 430 displays various types of data and information. For example, the display 430 displays a graphical user interface (GUI) or the like for receiving various operations from a user. The display 430 includes, for example, a liquid crystal display, a cathode ray tube (CRT) display, or the like.

The input interface 450 receives various instructions from an operator. The input interface 450 is, for example, a mouse, a keyboard, a panel switch, a slider switch, a trackball, a rotary encoder, an operation panel, and a touch panel. The input interface 450 is connected to the processing circuitry 490 via, for example, a bus, converts an operation instruction input from the operator into an electric signal, and outputs the electric signal to the processing circuitry 490. The input interface 450 is not limited to physical operating components such as a mouse and a keyboard. For example, circuitry that receives an electric signal corresponding to an operation instruction input from an external input device provided separately from the medical image processing apparatus 400 and outputs the electric signal to the processing circuitry 490 is also included in an example of an input interface.

The communication interface 470 implements various types of information communication protocols corresponding to the form of the in-hospital network NW. The communication interface 470 implements communication with other devices via the in-hospital network NW according to various protocols. The medical image processing apparatus 400 is connected to the in-hospital network NW via the communication interface 470, and communication with the medical image capturing apparatus 500 is implemented.

The processing circuitry 490 is, for example, a processor that functions as a center of the medical image processing apparatus 400. The processing circuitry 490 executes a program stored in the storage 410 to implement a function corresponding to the program. The processing circuitry 490 has an acquisition function 491, a first rendering image generation function 492, a second rendering image generation function 493, a base colorization function 494, a shadow colorization function 495, a third rendering image generation function 496, a display control function 497, an attenuation transfer function setting function 498, and a reflection transfer function setting function 499. Here, for example, the respective processing functions of the acquisition function 491, the first rendering image generation function 492, the second rendering image generation function 493, the base colorization function 494, the shadow colorization function 495, the third rendering image generation function 496, the display control function 497, the attenuation transfer function setting function 498, and the reflection transfer function setting function 499, which are components of the processing circuitry 490 illustrated in FIG. 23, are recorded in the storage 410 in the form of a program executable by a computer. The processing circuitry 490 reads each program from the storage 410 and executes each read program to implement a function corresponding to each program. In other words, the processing circuitry 490 in a state of reading each program has the functions of the processing circuitry 490 illustrated in FIG. 23. The processing circuitry 490 is implemented by, for example, a processor. The acquisition function 491 is an example of an acquisition unit. The first rendering image generation function 492 is an example of a first acquisition unit. The second rendering image generation function 493 is an example of a second acquisition unit. The base colorization function 494 is an example of a color image generation unit. The shadow colorization function 495 is an example of the color image generation unit. The third rendering image generation function 496 is an example of a generation unit. The display control function 497 is an example of a display control unit. The attenuation transfer function setting function 498 is an example of an attenuation transfer function setting unit. The reflection transfer function setting function 499 is an example of a reflection transfer function setting unit.

The acquisition function 491, the first rendering image generation function 492, the second rendering image generation function 493, the base colorization function 494, the shadow colorization function 495, the third rendering image generation function 496, the display control function 497, the attenuation transfer function setting function 498, and the reflection transfer function setting function 499 respectively have substantially the same functions as, for example, the acquisition function 382, the first rendering image generation function 383, the second rendering image generation function 384, the base colorization function 385, the shadow colorization function 386, the third rendering image generation function 387, the display control function 388, the attenuation transfer function setting function 389, and the reflection transfer function setting function 3810 in the first embodiment.

Thus, the medical image processing apparatus 400 according to the fourth embodiment can be expected to have similar effects to those in the first embodiment.

The medical image processing apparatus 400 according to the fourth embodiment is connected to the medical image capturing apparatus 500 via the in-hospital network NW, but the connection between the medical image processing apparatus 400 and the medical image capturing apparatus 500 is not limited to the in-hospital network. That is, the form of the network is arbitrary, and may be, for example, a wireless/wired LAN such as a hospital backbone local area network (LAN), an Internet network, a telecommunication circuit network, an optical fiber communication network, a cable communication network, a satellite communication network, or the like.

### [Fifth Embodiment]

In the ultrasonic diagnostic apparatuses 1 according to the first to third embodiments and the medical image processing apparatus 400 according to the fourth embodiment described above, coefficients based on a surface rendering image or a light attenuation image generated outside the ultrasonic diagnostic apparatus 1 or the medical image processing apparatus 400 may be acquired. A case in which this modification is applied to the first embodiment will be referred to as a fifth embodiment, and portions different from those of the above-described first embodiment will be described. This modification can also be applied to the second to fourth embodiments.

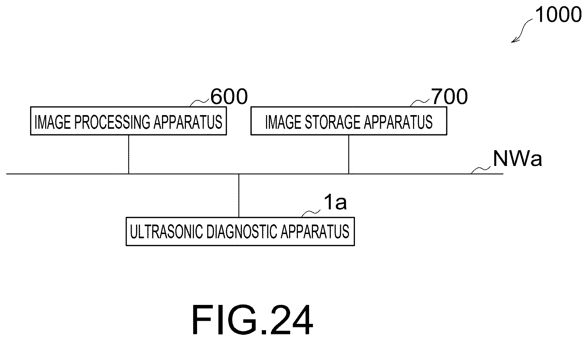

FIG. 24 is a block diagram illustrating an example of a configuration of an ultrasonic diagnostic system according to the fifth embodiment. As illustrated in FIG. 24, an ultrasonic diagnostic system 1000 includes an ultrasonic diagnostic apparatus 1a, an image processing apparatus 600, and an image storage apparatus 700. As illustrated in FIG. 24, the ultrasonic diagnostic apparatus 1a, the image processing apparatus 600, and the image storage apparatus 700 are communicatively connected via a network NWa. The configuration illustrated in FIG. 24 is merely an example, and various devices such as terminal devices may be connected to the network NWa.

The image processing apparatus 600 acquires B mode data and Doppler data from the ultrasonic diagnostic apparatus 1a via the network NWa, and generates three-dimensional ultrasonic data. Specifically, the image processing apparatus 600 generates ultrasonic volume data as three-dimensional ultrasonic data. The image processing apparatus 600 generates an image obtained by executing surface rendering on the three-dimensional ultrasonic data. The image processing apparatus 600 transmits the generated image having been subjected to the surface rendering to the ultrasonic diagnostic apparatus 1a or the image storage apparatus 700 via the network NWa. The "surface rendering" and the above-described "surface rendering processing" have the same meaning. In the following description, the expression "surface rendering" may be used instead of the expression "surface rendering processing".

The image processing apparatus 600 obtains a light attenuation coefficient. Specifically, the image processing apparatus 600 executes photon mapping processing as global illumination on the ultrasonic volume data, and generates a photon map that is a map regarding attenuation of light generated by executing the photon mapping processing. Next, the image processing apparatus 600 generates a light attenuation image based on the photon map. Then, the image processing apparatus 600 converts the light attenuation image into a light attenuation coefficient by applying a coefficient conversion transfer function to the light attenuation image. The image processing apparatus 600 transmits the light attenuation coefficient to the ultrasonic diagnostic apparatus 1a or the image storage apparatus 700 via the network NWa. The "global illumination" and the above-described "global illumination rendering processing" have the same meaning. In the following description, the expression "global illumination" may be used instead of the expression "global illumination rendering processing". The "light attenuation image" and the above-described "global illumination rendering image" have the same meaning. In the following description, the expression of the light attenuation image may be used instead of the global illumination rendering image.

The image storage apparatus 700 stores ultrasonic data generated by the ultrasonic diagnostic apparatus 1a, various types of data generated by the image processing apparatus 600, and the like. The image storage apparatus 700 transmits stored ultrasonic data, various types of data, and the like to the ultrasonic diagnostic apparatus 1a or the image processing apparatus 600 via the network NWa. For example, the image storage apparatus 700 is an image server such as a picture archiving and communication system (PACS). The image storage apparatus 700 may be implemented by a server group (cloud) connected to the ultrasonic diagnostic system 1000 via a network.

The ultrasonic diagnostic apparatus 1a generates ultrasonic data from a reflected wave signal obtained by transmitting and receiving ultrasonic waves. Then, the ultrasonic diagnostic apparatus 1 transmits the generated ultrasonic data to the image processing apparatus 600 and the image storage apparatus 700 via the network NWa.

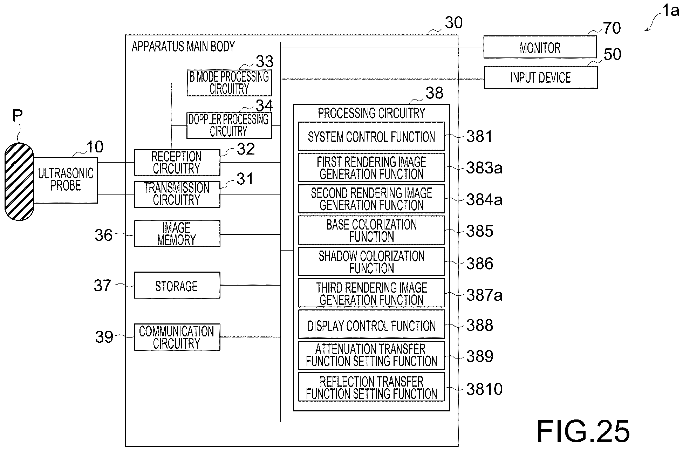

FIG. 25 is a diagram illustrating an example of the configuration of the ultrasonic diagnostic apparatus 1a according to the fifth embodiment, and is a diagram corresponding to FIG. 1. As illustrated in FIG. 25, the ultrasonic diagnostic apparatus 1a according to the fifth embodiment is configured by adding communication circuitry 39 to the ultrasonic diagnostic apparatus 1 according to the first embodiment described above. The ultrasonic diagnostic apparatus 1a according to the fifth embodiment is different from the ultrasonic diagnostic apparatus 1 according to the first embodiment described above in that the image generation circuitry 35 and the acquisition function 382 are not provided. Furthermore, since functions of a first rendering image generation function, a second rendering image generation function, and a third rendering image generation function are different from those in the first embodiment, the functions are referred to as a first rendering image generation function 383a, a second rendering image generation function 384a, and a third rendering image generation function 385a. The first rendering image generation function 383a, the second rendering image generation function 384a, and the third rendering image generation function 385a correspond to a first acquisition unit, a second acquisition unit, and a generation unit in the present embodiment, respectively.

The communication circuitry 39 is loaded with various information communication protocols according to the form of the network NWa. The communication circuitry 39 implements communication with other devices via the network NWa according to the various protocols.

The first rendering image generation function 383a according to the fifth embodiment acquires a surface rendering image, whereas the first rendering image generation function 383 according to the first embodiment described above generates and acquires a surface rendering image by executing surface rendering. The surface rendering image is an example of a first image on which surface rendering has been executed.

The second rendering image generation function 384a according to the fifth embodiment acquires a light attenuation coefficient, whereas the second rendering image generation function 384 according to the first embodiment described above generates and acquires a light attenuation image by executing global illumination. The light attenuation image is an example of a second image on which global illumination has been executed.

The third rendering image generation function 387a according to the fifth embodiment generates a composite image based on an image having been subjected to surface rendering and a light attenuation coefficient, whereas the third rendering image generation function 387 according to the first embodiment described above generates and acquires a rendering image based on a surface rendering image and a light attenuation coefficient based on a light attenuation image. The composite image is an example of a rendering image.

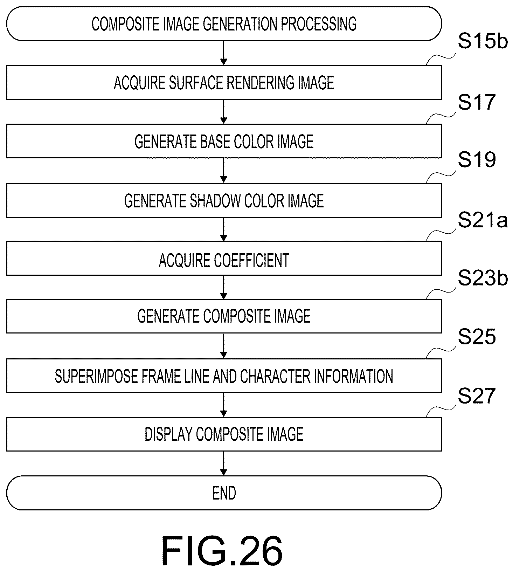

FIG. 26 is a flowchart for describing an example of composite image generation processing executed in the ultrasonic diagnostic apparatus 1a according to the fifth embodiment, and is a diagram corresponding to FIG. 3. In the composite image generation processing according to the fifth embodiment, a surface rendering image is acquired, a base color image and a shadow color image are generated as a plurality of color images, a light attenuation coefficient is acquired, a composite image is generated, a frame line or character information is superimposed, and a composite image is displayed. For example, the composite image generation processing is processing executed when the surface rendering data is acquired.

As illustrated in FIG. 26, first, the first rendering image generation function 383a in the processing circuitry 38 of the apparatus main body 30 acquires a surface rendering image (step S15b). Specifically, the first rendering image generation function 383a acquires an image having been subjected to the surface rendering from the image memory 36 or from the image processing apparatus 600 or the image storage apparatus 700 via the communication circuitry 39 and the network NWa. Processing of steps S17 and S19 after step S15b is similar to that in FIG. 3, and thus the description thereof is omitted.

Next, as illustrated in FIG. 26, the second rendering image generation function 384a in the processing circuitry 38 of the apparatus main body 30 acquires a coefficient (step S21a). Specifically, the second rendering image generation function 384a acquires a light attenuation coefficient from the image memory 36 or from the image processing apparatus 600 or the image storage apparatus 700 via the communication circuitry 39 and the network NWa.

Next, as illustrated in FIG. 26, the third rendering image generation function 387a in the processing circuitry 38 of the apparatus main body 30 generates a composite image (step S23b). Specifically, the third rendering image generation function 387a generates a composite image by combining the base color image generated in step S17 and the shadow color image generated in step S19 as a plurality of color images, based on the light attenuation coefficient acquired in step S21. The content of more specific processing of step S23b is similar to that in the first embodiment, and thus the description thereof is omitted. Processing of steps S25 and S27 after step S23b is similar to that in FIG. 3, and thus the description thereof is omitted. In step S27, the composite image generation processing ends by displaying the composite image.

As described above, since the ultrasonic diagnostic apparatus 1a according to the fifth embodiment executes various types of image processing in the image processing apparatus 600 and generates a base color image, a shadow color image, and a composite image, processing load of the ultrasonic diagnostic apparatus 1a can be reduced.

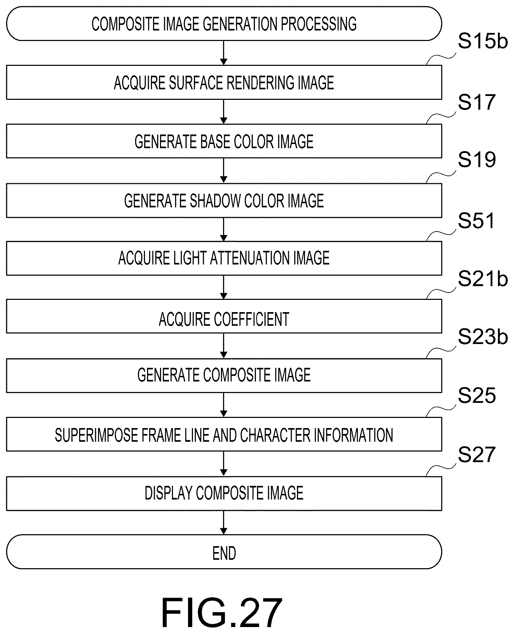

The ultrasonic diagnostic apparatus 1a according to the fifth embodiment described above may acquire a light attenuation image and acquire a light attenuation coefficient based on the light attenuation image. FIG. 27 is a diagram illustrating another example of the composite image generation processing executed in the ultrasonic diagnostic apparatus 1a according to the fifth embodiment, and is a diagram corresponding to FIG. 26. Processing of step S15b is similar to that in FIG. 26, and thus the description thereof will be omitted. Processing of steps S17 and S19 after step S15b is similar to that in FIG. 3, and thus the description thereof is omitted.

Next, as illustrated in FIG. 27, the second rendering image generation function 384a in the processing circuitry 38 of the apparatus main body 30 acquires a light attenuation image (step S51). Specifically, the second rendering image generation function 384a acquires a light attenuation image from the image memory 36 or from the image processing apparatus 600 or the image storage apparatus 700 via the communication circuitry 39 and the network NWa. More specifically, the second rendering image generation function 384a acquires a light attenuation image based on a photon map from the image memory 36 or from the image processing apparatus 600 or the image storage apparatus 700 via the communication circuitry 39 and the network NWa.

In step S51, the second rendering image generation function 384a may acquire a light attenuation image based on a reflection transfer function together with the photon map. In step S51, the second rendering image generation function 384a may acquire a light attenuation image by acquiring a photon map generated by executing photon mapping processing on ultrasonic volume data in the image processing apparatus 600 and generating a light attenuation image based on the acquired photon map. Naturally, the second rendering image generation function 384a may acquire a reflection transfer function together with a photon map generated by executing the photon mapping processing on the ultrasonic volume data in the image processing apparatus 600, and acquire a light attenuation image by generating a light attenuation image based on the acquired photon map and reflection transfer function.

Next, as illustrated in FIG. 27, the second rendering image generation function 384a acquires a coefficient (step S21b). Specifically, the second rendering image generation function 384a acquires a light attenuation coefficient based on the light attenuation image acquired in step S51. More specifically, the second rendering image generation function 384a applies a coefficient conversion transfer function for converting a light attenuation image into a coefficient to the light attenuation image, thereby converting the light attenuation image into a light attenuation coefficient to acquire the light attenuation coefficient. That is, the processing of step S21b illustrated in FIG. 27 is the same as the processing of step S21 illustrated in FIG. 3. Processing of step S23b is similar to that in FIG. 26, and thus the description thereof will be omitted. Processing of steps S25 and S27 after step S23b is similar to that in FIG. 3, and thus the description thereof is omitted. In step S27, the composite image generation processing ends by displaying the composite image.

### [Modification of First to Fifth Embodiments]

In the above-described first to fifth embodiments, the ultrasonic volume data is converted into voxel data of the Cartesian coordinate system, but the ultrasonic volume data may be used as raw data (Raw Data) without being converted into the Cartesian coordinate system.

In the first, second, fourth, and fifth embodiments described above, the data buffer is stored in the R channel, and the shadow buffer is stored in the G channel. However, the present invention is not limited thereto. The data buffer may be stored in the G channel or the B channel, and the shadow buffer may be stored in the R channel or the B channel. Similarly, also in the third embodiment described above, the data buffer may be stored in the G channel or the B channel, the shadow buffer may be stored in the R channel or the B channel, and the depth buffer may be stored in the R channel or the G channel.

In the first to fifth embodiments described above, a combination algorithm for combining a base color image and a shadow color image may be selected from a plurality of algorithms. Specifically, the storage 37 may store a plurality of combination algorithms, and the third rendering image generation function 387 may select a combination algorithm to be used from among the plurality of combination algorithms stored in a combination algorithm memory in accordance with input information input by the user and/or preset information that has been preset, and may combine a first color image and a second color image by using the selected combination algorithm together with a coefficient based on an image having been subjected to global illumination. The storage 37 that stores a plurality of combination algorithms is an example of a combination algorithm memory.

In the first to fifth embodiments described above, a case has been described in which two types of color maps of a base color map and a shadow color map or three types of color maps of a base color map, a shadow color map, and a depth color map are applied to the image having been subjected to surface rendering to generate two types of color images of a base color image and a shadow color image or three types of color images of a base color image, a shadow color image, and a depth color image, but the number of color maps applied to the image having been subjected to the surface rendering and the number of color images generated by applying the color maps are not limited thereto. That is, the number of color maps applied to the image having been subjected to the surface rendering and the number of color images generated by applying the color maps are arbitrary, and four or more types of color images may be generated by applying four or more types of color maps to the image having been subjected to the surface rendering. Then, the third rendering image generation functions 387 and 387a may generate a composite image by combining a plurality of color images based on a light attenuation coefficient.

Note that the word "processor" used in above descriptions means circuits such as, for example, a Central Processing Unit (CPU), a Graphics Processing Unit (GPU), an Application Specific Integrated Circuit (ASIC), a programmable logic device (for example, a Simple Programmable Logic Apparatus (SPLD), a Complex Programmable Logic Apparatus (CPLD), and a Field Programmable Gate Array (FPGA)). The processor executes functions by reading and executing programs stored in the storage 37. Note that programs may be configured to be directly integrated in the processor instead of being storing in the storage 37. In this case, the processor realizes functions by reading and executing programs stored in the circuitry. Note that the processor is not limited to the case arranged as a single processor circuit, but may be configured as a single processor by combining a plurality of independent circuits to realize functions. Furthermore, a plurality of component elements in Fig.1 may be integrated into one processor to realize the functions.

According to at least one embodiment described above, it is possible to generate a rendering image capable of securing visibility of a shadow portion while imparting a shadow effect.

While certain embodiments have been described, these embodiments have been presented by way of example only and are not intended to limit the scope of the inventions. The embodiments may be in a variety of other forms. Furthermore, various omissions, substitutions and changes may be made without departing from the scope of the inventions as defined by the appended claims. The embodiments and their modifications are included in the scope and the subject matter of the invention, and at the same time included in the scope of the claimed inventions.

## Claims

1. An ultrasonic diagnostic apparatus comprising
processing circuitry configured to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.

2. The ultrasonic diagnostic apparatus of claim 1, wherein the processing circuitry is further configured to acquire a second image on which global illumination has been executed on the three-dimensional ultrasonic data and acquire the light attenuation coefficient based on the second image.

3. The ultrasonic diagnostic apparatus of claim 1,
wherein the processing circuitry is further configured to:
generate a plurality of color images by applying a plurality of color maps to the first image subjected to the surface rendering; and
generate the rendering image by combining the plurality of color images based on the light attenuation coefficient.

4. The ultrasonic diagnostic apparatus of claim 3,
wherein the processing circuitry is further configured to:
generate a first color image by applying a first color map to the first image;
generate a second color image by applying a second color map different from the first color map to the first image; and
generate the rendering image by combining the first color image and the second color image using the light attenuation coefficient.

5. The ultrasonic diagnostic apparatus of claim 4, further comprising
a combination algorithm memory configured to store a plurality of combination algorithms for combining the first color image and the second color image,
wherein the processing circuitry is further configured to select a combination algorithm to be used from among the plurality of combination algorithms stored in the combination algorithm memory in accordance with input information input by a user and/or preset information that has been preset, and combine the first color image and the second color image by using the selected combination algorithm together with the light attenuation coefficient.

6. The ultrasonic diagnostic apparatus of claim 4, wherein the processing circuitry is further configured to combine the first color image and the second color image by linear interpolation using the light attenuation coefficient.

7. The ultrasonic diagnostic apparatus of claim 2,
wherein the processing circuitry is further configured to:
execute photon mapping processing as the global illumination on the three-dimensional ultrasonic data; and
acquire the second image based on a photon map that is a map related to attenuation of light generated by executing the photon mapping processing.

8. The ultrasonic diagnostic apparatus of claim 7,
wherein the processing circuitry is further configured to:
set an attenuation transfer function for determining a degree of attenuation of light; and
acquire the photon map using the attenuation transfer function in the photon mapping processing.

9. The ultrasonic diagnostic apparatus of claim 7,
wherein the processing circuitry is further configured to:
set a reflection transfer function for determining a degree of reflection of light by determining luminance and/or color; and
acquire the second image based on the reflection transfer function together with the photon map.

10. The ultrasonic diagnostic apparatus of claim 1, wherein the processing circuitry is further configured to execute color depth cueing processing on the first image.

11. The ultrasonic diagnostic apparatus of claim 1, wherein the processing circuitry is further configured to control a display unit to display the rendering image.

12. The ultrasonic diagnostic apparatus of claim 2, wherein the processing circuitry is further configured to apply, to the second image, a coefficient conversion transfer function for converting the second image into the light attenuation coefficient to convert the second image into the light attenuation coefficient.

13. The ultrasonic diagnostic apparatus of claim 1, further comprising
a texture memory configured to store a texture having a plurality of channels,
wherein the texture is configured to be able to store image data based on three-dimensional ultrasonic data in each of the plurality of channels.

14. A medical image processing apparatus comprising
processing circuitry configured to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.

15. A program causing a computer to:
acquire a first image on which surface rendering has been executed on three-dimensional ultrasonic data related to a subject;
acquire a light attenuation coefficient corresponding to the three-dimensional ultrasonic data; and
generate a rendering image based on the first image and the light attenuation coefficient.
